(19) Europäisches Patentamt
European Patent Office
Office européen des brevets

(11) EP 1 518 534 B1

(12) **EUROPEAN PATENT SPECIFICATION**

(45) Date of publication and mention of the grant of the patent:
**14.07.2010 Bulletin 2010/28**

(51) Int Cl.:
***A61K 8/81*** *(2006.01)* ***A61K 8/90*** *(2006.01)*
***A61K 8/92*** *(2006.01)* ***A61Q 1/04*** *(2006.01)*
***A61Q 1/10*** *(2006.01)* ***A61Q 3/02*** *(2006.01)*

(21) Application number: **04292148.6**

(22) Date of filing: **07.09.2004**

(54) **Bilayered cosmetic product, uses thereof and kit-of-parts containing this product.**

Zweischichtiges kosmetisches Produkt , dessen Verwendungen und Kit für das Schminken, das dieses Produkt enthält

Produit cosmétique bicouche, ses utilisations et kit de maquillage contenant ce produit

(84) Designated Contracting States:
**AT BE BG CH CY CZ DE DK EE ES FI FR GB GR HU IE IT LI LU MC NL PL PT RO SE SI SK TR**

(30) Priority: **26.09.2003 FR 0311340**

(43) Date of publication of application:
**30.03.2005 Bulletin 2005/13**

(73) Proprietor: **L'Oréal**
**75008 Paris (FR)**

(72) Inventors:
- **Blin, Xavier**
  **75015 Paris (FR)**
- **Ferrari, Véronique**
  **94700 Maisons-Alfort (FR)**

(74) Representative: **Boulard, Denis**
**L'Oréal**
**D.I.P.I.**
**25-29 Quai Aulagnier**
**92600 Asnières (FR)**

(56) References cited:
**EP-A- 1 440 680** **WO-A-01/89470**
**WO-A-03/046032** **US-A- 6 106 820**
**US-B1- 6 391 964**

- **A. BUZIN ET AL.: "Calorimetric study of block-copolymers of poly(n-butyl acrylate) and gradient poly(n-butyl acrylate-co-methyl-methacrylate)" POLYMER, vol. 43, 2002, pages 5563-5569, XP004374470**
- **LI I Q ET AL: "BLOCK COPOLYMER PREPARATION USING SEQUENTIAL NORMAL/ LIVING RADICAL POLYMERIZATION TECHNIQUES" MACROMOLECULES, ACS, WASHINGTON, DC, US, vol. 30, no. 18, 8 September 1997 (1997-09-08), pages 5195-5199, XP000698620 ISSN: 0024-9297**
- **DATABASE CA CHEMICAL ABSTRACTS SERVICE, COLUMBUS, OHIO, US; 9 January 2003 (2003-01-09), retrieved from STN Database accession no. 138:25256 & J. GOMEZ RIBELLES ET AL.: "Glass transition in homogeneous and heterogeneous interpenetrating polymer networks and its relation to concentration fluctuations." JOURNAL OF NON-CRYSTALLINE SOLIDS, 2002,**
- **DATABASE CA CHEMICAL ABSTRACTS SERVICE, COLUMBUS, OHIO, US; 9 July 1994 (1994-07-09), retrieved from stn Database accession no. 121:17699 & J. SHIOZAWA ET AL.: "Applications of optically arranged metal-acrylic films for super-covering makeups" JOURNAL OF SCCJ, vol. 27, no. 3, 1993, pages 326-337, JP**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

The present invention relates to a cosmetic product comprising at least two compositions that may be applied successively to the skin either of the face or of the body, the lower and upper eyelids, the lips and integuments such as the nails, the eyebrows, the eyelashes or the hair. The present invention also relates to a process for making up the face and the body using these two compositions.

Each composition may be a foundation, a makeup rouge, an eyeshadow, a concealer product, a blusher, a free or compacted powder, a lipstick, a lip balm, a lip gloss, a lip pencil, an eye pencil, a mascara, an eyeliner, a nail varnish or a body-makeup or skin-colouring product.

The known compositions have poor staying power over time and in particular poor colour fastness and poor staying power of the gloss over time. This poor staying power may be characterized by a modification of the colour (colour change or fading) generally following an interaction with the sebum and/or sweat secreted by the skin in the case of foundations and makeup powders, or an interaction with the saliva in the case of lipsticks, or a reduction in the gloss, in the case of a nail varnish. This obliges the user to reapply the makeup very often, which may constitute a loss of time.

In addition, the known compositions have a tendency to migrate, i.e. to travel over time in the folds of the wrinkles and fine lines of the skin, especially those around the lips and the eyes, resulting in an unattractive effect. Women often mention this migration as a major defect of cosmetic compositions, in particular standard lipsticks and eyeshadows. The term "migration" means an overflowing of the composition and in particular of the colour, beyond the initial line of the makeup.

"Transfer-resistant" lip and skin makeup compositions are compositions that have the advantage of forming a deposit that does not come off, at least partly, onto the supports with which they are placed in contact (glass, clothing, cigarette or fabrics).

The known transfer-resistant compositions are generally based on silicone resins and volatile silicone oils, and, although having improved staying power properties, they have the drawback of leaving on the skin and the lips, after evaporation of the volatile silicone oils, a film that becomes uncomfortable over time (sensation of dryness and tautness), which puts a certain number of women off lipsticks of this type.

In addition, these compositions based on volatile silicone oils and silicone resins lead to matt coloured films. Now, women are nowadays in search of products, especially for colouring the lips or the eyelids, that are glossy while at the same time having good staying power and being transfer-resistant.

To obtain transfer-resistant products with good staying power, volatile oils or polymers dispersed in a volatile solvent are often used. However, these products are not sufficiently glossy. A cosmetic product comprising two compositions, a first containing a polymer dispersed in a volatile solvent, over which is applied a second glossy and greasy composition, has thus been envisaged (FR-A-2 823 101). The staying power of the colour of the makeup obtained with these compositions is good, but the disappearance of the second composition in the course of the day when it is worn results in a loss of gloss, which is desired by the consumer.

The company Kose has moreover proposed in its patent application JP-A-05 221 829 to use a gel based on perfluoro materials, which is applied over a film of lipstick so as to prevent it from being transferred onto other surfaces, the gel being incompatible with the film of lipstick.

Mention may also be made of patent application WO-A-97/17057, which describes a method for increasing the staying-power and transfer-resistance properties, consisting in applying two compositions one over the other. The composition to be applied has a global Hildebrand solubility parameter of less than 8.5 $(cal/cm^3)^{1/2}$, and the composition to be applied as topcoat must contain oils whose calculated coefficient of partition ClogP is at least equal to 13.

Patent US-A-6 001 374 proposes a multicoat makeup system, which consists in using a composition containing a resin that is soluble in alcohol and insoluble in water, which may be applied as a basecoat or as a topcoat, and which has the advantage of not marking an object placed in contact with the makeup and of being resistant to water and to rubbing, while at the same time having a certain level of gloss. However, this composition contains a water-soluble alcohol, in particular ethanol, which has an irritant, dehydrating nature on the skin and more especially on the lips, and which is particularly uncomfortable when the skin or the lips are damaged.

Patent application WO 02/067877 describes a method for improving the aesthetic properties of a transfer-resistant composition, which consists in applying a second composition over a film of transfer-resistant composition. The second composition should not interact chemically with the transfer-resistant composition, so as not to impair its cosmetic properties. Some of the products described in the said document have an unpleasant odour and are tacky. Other products are not glossy enough.

The aim of the present invention is to propose a novel route for formulating a cosmetic product, in particular a makeup product, of the type comprising two compositions to be applied successively one after the other. propose a novel route for formulating a cosmetic product, in particular a makeup product, of the type comprising two compositions to be applied successively one after the other.

An object of the invention is in particular to prepare such a cosmetic product that allows good transfer-resistance

and gloss fastness properties, compared especially with the product of patent application WO-A-97/17057.

Patent applications EP 1 440 680 and WO 03/046032 and document A. Buzin et Al. "calorimetric study of block-copolymer of poly(n-butyl acrylate) and gradient poly(n-butyl acrylate-co-methyl-methacrylate)" Polymer vol. 43, 2002 pages 5563-5569 describe cosmetic compositions comprising linear block ethylene copolymers, having at least two blocks with different glass transition temperatures, and prepared by living polymerization.

Patent application WO 03/046032 also describes linear block ethylene copolymers, having at least two blocks with different glass transition temperatures and which can be prepared by anionic polymerization, free radical polymerization or preferably by controlled radical polymerization. This application describes their use for enhancing the staying power of the composition and gives an example of a nail coating comprising a first composition comprising such a copolymer and a second copolymer.

An object of the present invention is also to propose a cosmetic product, in particular a makeup product, that simultaneously combines the following properties: "transfer resistance", migration resistance, colourfastness, comfort, absence of dehydration, gloss and staying power of the gloss over time.

The Applicant has found that these objects can be obtained by combining a first composition comprising, in a cosmetically acceptable organic liquid medium, at least one film-forming ethylenic block polymer, wherein said block polymer comprises at least one first block and at least one second block that have different glass transition temperatures (Tg), wherein the first block of the polymer is chosen from:

- a) a block with a Tg of greater than or equal to 40°C,
- b) a block with a Tg of less than or equal to 20°C,
- c) a block with a Tg of between 20 and 40°C, and

the second block is chosen from a category a), b) or c) different from the first block, and wherein the first and second blocks are linked together via an intermediate segment, which is a random polymer, comprising at least one constituent monomer of the first block and at least one constituent monomer of the second block, and wherein said block polymer has a polydispersity index I of greater than 2,

and a second composition, which is different from the first composition, comprising a cosmetically acceptable medium.

Thus, the compositions according to at least some of the aspects of the invention make it possible , to obtain a cosmetic result that is very glossy on application and over time, which does not migrate, does not transfer and shows good staying power, while at the same time being comfortable on application and over time (non-tacky and non-dehydrating, and no tautness).

In particular, the product of the invention makes it possible to obtain non-tacky continuous deposits on the skin or the lips, which give good coverage, having a very glossy appearance, which is adapted to the desire of consumers, does not migrate, does not transfer, has good staying power, is not oily and does not dry out the skin, the hair or the lips onto which it is applied, either during application or over time. It also has good stability properties and thus allows a uniform and aesthetic application.

It has moreover been found that the compositions of the product according to the invention have particularly advantageous spreading and adhesion properties on the skin, the lips or the eyelashes, and also a creamy and pleasant feel.

These staying-power, transfer-resistant and migration-resistant properties, allied with the glossy and non-greasy appearance, make a product that is particularly suitable for producing makeup products for the lips such as lipstick and lip glosses, for the eyes, such as mascara, eyeliners and eyeshadows, and for the nails or the hair.

One subject of the present invention is thus a cosmetic product for application to the skin, especially the skin of the face or the neck, the lips or the eyelids, comprising a first and a second composition, the first composition containing, in a cosmetically acceptable organic liquid medium, at least one film-forming linear ethylenic block polymer, and the second composition, which is different from the

Preferably, the block polymer is free of styrene units.

Preferably also, the block polymer is non-elastomeric.

Another subject of the present invention is a cosmetic product for application to the integuments, especially the nails, the hair or the eyebrows, comprising a first and a second composition, the first composition containing, in a cosmetically acceptable organic liquid medium, at least one film-forming linear ethylenic block polymer, and the second composition, which is separate from the first, comprising a cosmetically acceptable medium.

The product of the invention is in particular a makeup product for the skin, the nails or the hair.

The term "makeup product" means a product containing a colouring agent allowing the deposition of a colour onto a keratin material (the skin or the integuments) of a human being by applying onto the keratin material products such as lipsticks, makeup powders, eyeliners, foundations, self-tanning agents or semi-permanent makeup products (tattoos).

The product according to the invention comprises at least two cosmetically acceptable compositions packaged

separately or together in the same packaging article or in at least two separate packaging articles.

Preferably, these compositions are packaged separately and, advantageously, in separate packaging articles.

One subject of the present invention is thus, in particular, a cosmetic makeup product in the form of a mascara, a makeup rouge, an eyeshadow, a lipstick, a nail varnish, a product especially having care properties, a mascara, an eyeliner, a concealer product or a makeup product for the body (such as a tattoo) or the hair.

A subject of the invention is also a makeup kit containing a cosmetic makeup product as defined above, in which the various compositions are packaged separately and are advantageously accompanied by suitable application means. These means may be fine brushes, coarse brushes, pens, pencils, felts, quills, sponges, tubes and/or foam tips.

The first composition of the product according to the invention may constitute a base coat applied onto the keratin material, and the second composition a topcoat. It is possible, however, to apply under the first coat an undercoat that may or may not have the constitution of the second coat.

It is also possible to deposit an overcoat onto the second coat, which may or may not have an identical constitution to that of the first coat. Preferably, the makeup obtained is a two-coat makeup.

The second composition may also constitute a basecoat applied onto the keratin material, and the first composition a topcoat.

In particular, the base coat is a lipstick, a mascara, a lip gloss, an eyeliner, a nail varnish, a nailcare product or a body makeup product, and the top coat a care or protective product.

The invention also relates to a process for making up the skin and/or the lips and/or the integuments, which consists in applying a cosmetic product as defined above to the skin and/or the lips and/or the integuments.

A subject of the invention is also a cosmetic care or makeup process for human skin and/or lips and/or integuments, which consists in applying to the skin, the lips and/or the integuments a first coat of a first composition comprising, in a cosmetically acceptable organic liquid medium, at least one film-forming linear ethylenic block polymer, and then in applying, over all or part of the first coat, a second coat of a second composition comprising a cosmetically acceptable medium.

This process may consist in applying to human skin, lips and/or integuments a first coat of a first composition comprising, in a cosmetically acceptable organic liquid medium, at least one film-forming linear ethylenic block polymer, leaving the said first coat to dry, and then applying, over all or part of the first coat, a second coat of a second composition comprising a cosmetically acceptable medium.

This process may also consist in applying to human skin, lips and/or integuments a first coat of a composition comprising a cosmetically acceptable medium, leaving the said first coat to dry, and then applying, over all or part of the first coat, a second coat of a composition comprising, in a cosmetically acceptable organic liquid medium, at least one film-forming linear ethylenic block polymer.

The product according to the invention may be applied not only to the face but also the scalp and the body, the lips, the inside of the lower eyelids, and integuments, for instance the nails, the eyelashes, the hair, the eyebrows, or even other body hairs. The second composition may form units and may be applied with a pen, a pencil or any other instrument (sponge, finger, fine brush, coarse brush or quill). This makeup may also be applied to makeup accessories, for instance false nails, false eyelashes, wigs or pastilles or patches that adhere to the skin or the lips (such as beauty spots).

A subject of the invention is also a made-up support, comprising a first coat of a first composition comprising a cosmetically acceptable first medium, and a second coat of a second composition comprising a cosmetically acceptable second medium, the said first coat being applied to the support first or over all or part of the second coat.

This support may in particular be a hairpiece such as a wig, false nails, false eyelashes, or patches that adhere to the skin or the lips (such as beauty spots).

The invention also relates to the cosmetic use of the cosmetic product defined above to improve the comfort properties, in particular in terms of the absence of tack and/or absence of dehydration, and/or the gloss and/or the transfer and/or the migration and/or the staying power of the makeup on the skin and/or the lips and/or the integuments.

Finally, a subject of the invention is the use of a cosmetic product comprising a first and a second composition, the first composition comprising, in a cosmetically acceptable organic liquid medium, at least one film-forming linear ethylenic block polymer as described below, and the second composition comprising a cosmetically acceptable medium, to give the skin and/or the lips and/or the integuments a comfortable and/or glossy and/or transfer-resistant and/or migration-resistant cosmetic result and/or a cosmetic result with good staying power.

## Block polymer of the first composition

The block polymer of the first composition of the product according to the invention is preferably a film-forming linear ethylenic block polymer.

The first polymer contains at least one block polymer. The term "block polymer" means a polymer comprising at least two different blocks and preferably at least three different blocks.

According to one embodiment, the block polymer of the first composition is an ethylenic polymer. The term "ethylenic polymer" means a polymer obtained by polymerizing monomers comprising an ethylenic unsaturation.

According to one embodiment, the block polymer of the first composition is a linear polymer. In contrast, a polymer of non-linear structure is, for example, a polymer of branched, starburst or grafted structure, or the like.

According to one embodiment, the block polymer of the first composition is a film-forming polymer. The term "film-forming polymer" means a polymer capable of forming, by itself or in the presence of an auxiliary film-forming agent, a continuous film that adheres to a support and especially to keratin materials.

According to one embodiment, the block polymer of the first composition is a non-elastomeric polymer.

The term "non-elastomeric polymer" means a polymer which, when it is subjected to a constraint intended to stretch it (for example by 30% relative to its initial length), does not return to a length substantially identical to its initial length when the constraint ceases.

More specifically, the term "non-elastomeric polymer" denotes a polymer with an instantaneous recovery Ri < 50% and a delayed recovery $R_{2h}$ < 70% after having been subjected to a 30% elongation. Preferably, $R_i$ is < 30% and $R_{2h}$ < 50%.

More specifically, the non-elastomeric nature of the polymer is determined according to the following protocol:

A polymer film is prepared by pouring a solution of the polymer in a Teflon-coated mould, followed by drying for 7 days in an environment conditioned at 23 $\pm$ 5°C and 50 $\pm$ 10% relative humidity.

A film about 100 $\mu$m thick is thus obtained, from which are cut rectangular specimens (for example using a punch) 15 mm wide and 80 mm long.

This sample is subjected to a tensile stress using a machine sold under the reference Zwick, under the same temperature and humidity conditions as for the drying.

The specimens are pulled at a speed of 50 mm/min and the distance between the jaws is 50 mm, which corresponds to the initial length ($l_0$) of the specimen.

The instantaneous recovery $R_i$ is determined in the following manner:

- the specimen is pulled by 30% ($\varepsilon_{max}$), i.e. about 0.3 times its initial length ($l_0$)
- the constraint is released by applying a return speed equal to the tensile speed, i.e. 50 mm/min, and the residual elongation of the specimen is measured as a percentage, after returning to zero constraint ($\varepsilon_1$).

The percentage instantaneous recovery ($R_i$) is given by the following formula:

$$R_i = (\varepsilon_{max} - \varepsilon_1)/\varepsilon_{max}) \times 100$$

To determine the delayed recovery, the percentage residual elongation of the specimen ($\varepsilon_{2h}$) is measured, 2 hours after returning to zero constraint.

The percentage delayed recovery ($R_{2h}$) is given by the following formula:

$$R_{2h} = (\varepsilon_{max} - \varepsilon_{2h})/\varepsilon_{max}) \times 100$$

Purely as a guide, a polymer according to one embodiment of the invention has an instantaneous recovery $R_i$ of 10% and a delayed recovery $R_{2h}$ of 30%.

According to another embodiment, the block polymer of the first composition does not comprise any styrene units. The expression "polymer free of styrene units" means a polymer comprising less than 10%, preferably less than 5%, preferentially less than 2% and more preferentially less than 1% by weight i) of styrene units of formula $-CH(C_6H_5)-CH_2-$ or ii) of substituted styrene units, for instance methylstyrene, chlorostyrene or chloromethylstyrene.

According to one embodiment, the block polymer of the first composition is derived from aliphatic ethylenic monomers. The term "aliphatic monomer" means a monomer comprising no aromatic groups.

According to one embodiment, the block polymer is an ethylenic polymer derived from aliphatic ethylenic monomers comprising a carbon-carbon double bond and at least one ester group -COO- or amide group -CON-. The ester group may be linked to one of the two unsaturated carbons via the carbon atom or the oxygen atom. The amide group may be linked to one of the two unsaturated carbons via the carbon atom or the nitrogen atom.

According to one embodiment, the block polymer comprises at least one first block and at least one second block.

The term "at least one block" means one or more blocks.

It is pointed out that, in the text hereinabove and hereinbelow, the terms "first" and "second" blocks do not in any way condition the order of the said blocks in the polymer structure.

According to one embodiment, the block polymer comprises at least one first block and at least one second block that have different glass transition temperatures (Tg).

In this embodiment, the first and second blocks may be linked together via an intermediate segment with a glass transition temperature between the glass transition temperatures of the first and second blocks.

According to one embodiment, the block polymer comprises at least one first block and at least one second block linked together via an intermediate segment comprising at least one constituent monomer of the first block and at least one constituent monomer of the second block.

Preferably, the intermediate block is derived essentially from constituent monomers of the first block and of the second block.

The term "essentially" means at least 85%, preferably at least 90%, better still 95% and even better still 100%.

Advantageously, the intermediate segment comprising at least one constituent monomer of the first block and at least one constituent monomer of the second block of the polymer is a random polymer.

According to one embodiment, the block polymer comprises at least one first block and at least one second block that are incompatible in the organic liquid medium of the composition of the invention.

The term "mutually incompatible blocks" means that the mixture formed from the polymer corresponding to the first block and of the polymer corresponding to the second block is not miscible in the organic liquid that is in major amount by weight contained in the organic liquid medium of the composition, at room temperature (25°C) and atmospheric pressure ($10^5$ Pa), for a content of the polymer mixture of greater than or equal to 5% by weight, relative to the total weight of the mixture (polymers and major organic liquid), it being understood that:

i) the said polymers are present in the mixture in a content such that the respective weight ratio ranges from 10/90 to 90/10, and
ii) each of the polymers corresponding to the first and second blocks has an average (weight-average or number-average) molecular mass equal to that of the block polymer ± 15%.

When the organic liquid medium comprises a mixture of organic liquids, in the case of two or more liquids present in identical mass proportions, the said polymer mixture is immiscible in at least one of them.

When the organic liquid medium comprises only one organic liquid, this liquid obviously constitutes the liquid that is in major amount by weight.

The term "organic liquid medium" means a medium containing at least one organic liquid, i.e. at least one organic compound that is liquid at room temperature (25°C) and atmospheric pressure ($10^{5 Pa}$). According to one embodiment, the major liquid of the organic liquid medium is a volatile or non-volatile oil (fatty substance). Preferably, the organic liquid is cosmetically acceptable (acceptable tolerance, toxicology and feel).

According to one embodiment, the major liquid of the organic liquid medium is the polymerization solvent or one of the polymerization solvents of the block polymer, as are described below.

The term "polymerization solvent" means a solvent or a mixture of solvents. The polymerization solvent may be chosen especially from ethyl acetate, butyl acetate, alcohols such as isopropanol and ethanol, aliphatic alkanes such as isododecane, and mixtures thereof. Preferably, the polymerization solvent is a mixture of butyl acetate and isopropanol, or isododecane.

In general, the block polymer may be incorporated into the composition to a high solids content, typically greater than 10%, greater than 20%, more preferably greater than 30% and more preferentially greater than 45% by weight relative to the total weight of the composition, while at the same time being easy to formulate.

Preferably, the block polymer comprises no silicon atoms in its skeleton. The term "skeleton" means the main chain of the polymer, as opposed to the pendent side chains.

Preferably, the polymer according to the invention is not water-soluble, i.e. the polymer is not soluble in water or in a mixture of water and linear or branched lower monoalcohols containing from 2 to 5 carbon atoms, for instance ethanol, isopropanol or n-propanol, without pH modification, at an active material content of at least 1% by weight, at room temperature (25°C).

According to one embodiment, the block polymer has a polydispersity index I of greater than 2.

Advantageously, the block polymer used in the compositions according to the invention has a polydispersity index I of greater than 2, for example ranging from 2 to 9, preferably greater than or equal to 2.5, for example ranging from 2.5 to 8 and better still greater than or equal to 2.8, and especially ranging from 2.8 to 6.

The polydispersity index I of the polymer is equal to the ratio of the weight-average mass Mw to the number-average mass Mn.

The weight-average molar mass (Mw) and number-average molar mass (Mn) are determined by gel permeation liquid chromatography (THF solvent, calibration curve established with linear polystyrene standards, refractometric detector).

The weight-average mass (Mw) of the block polymer is preferably less than or equal to 300 000; it ranges, for example, from 35 000 to 200 000 and better still from 45 000 to 150 000.

The number-average mass (Mn) of the block polymer is preferably less than or equal to 70 000; it ranges, for example, from 10 000 to 60 000 and better still from 12 000 to 50 000.

Each block of the block polymer is derived from one type of monomer or from several different types of monomer.

This means that each block may consist of a homopolymer or a copolymer; this copolymer constituting the block may in turn be random or alternating.

The glass transition temperatures indicated for the first and second blocks may be theoretical Tg values determined from the theoretical Tg values of the constituent monomers of each of the blocks, which may be found in a reference manual such as the Polymer Handbook, 3rd Edition, 1989, John Wiley, according to the following relationship, known as Fox's law:

$$1/Tg = \sum_i (\varpi_i / Tg_i),$$

$\omega_i$ being the mass fraction of the monomer i in the block under consideration and $Tg_i$ being the glass transition temperature of the homopolymer of the monomer i.

Unless otherwise indicated, the Tg values indicated for the first and second blocks in the present patent application are theoretical Tg values.

The difference between the glass transition temperatures of the first and second blocks is generally greater than 10°C, preferably greater than 20°C and better still greater than 30°C.

In particular, the block polymer comprises at least one first block and at least one second block such that the first block may be chosen from:

- a) a block with a Tg of greater than or equal to 40°C,
- b) a block with a Tg of less than or equal to 20°C,
- c) a block with a Tg of between 20 and 40°C, and the second block can be chosen from a category a), b) or c) different from the first block.

In the present invention, the expression:

"between ... and ..." is intended to denote a range of values for which the limits mentioned are excluded, and
"from ... to ..." and "ranging from ... to ..." are intended to denote a range of values for which the limits are included.

a) Block with a Tg of greater than or equal to 40°C

The block with a Tg of greater than or equal to 40°C has, for example, a Tg ranging from 40 to 150°C, preferably greater than or equal to 50°C, for example ranging from 50°C to 120°C and better still greater than or equal to 60°C, for example ranging from 60°C to 120°C.

The block with a Tg of greater than or equal to 40°C may be a homopolymer or a copolymer.
The block with a Tg of greater than or equal to 40°C may be totally or partially derived from one or more monomers, which are such that the homopolymer prepared from these monomers has a glass transition temperature of greater than or equal to 40°C.

In the case where this block is a homopolymer, it is derived from monomers which are such that the homopolymers prepared from these monomers have glass transition temperatures of greater than or equal to 40°C. This first block may be a homopolymer consisting of only one type of monomer (for which the Tg of the corresponding homopolymer is greater than or equal to 40°C).

In the case where the first block is a copolymer, it may be totally or partially derived from one or more monomers, the nature and concentration of which are chosen such that the Tg of the resulting copolymer is greater than or equal to 40°C. The copolymer may comprise, for example:

- monomers which are such that the homopolymers prepared from these monomers have Tg values of greater than or equal to 40°C, for example a Tg ranging from 40 to 150°C, preferably greater than or equal to 50°C, for example

ranging from 50°C to 120°C and better still greater than or equal to 60°C, for example ranging from 60°C to 120°C, and

- monomers which are such that the homopolymers prepared from these monomers have Tg values of less than 40°C, chosen from monomers with a Tg of between 20 and 40°C and/or monomers with a Tg of less than or equal to 20°C, for example a Tg ranging from -100 to 20°C, preferably less than 15°C, especially ranging from -80°C to 15°C and better still less than 10°C, for example ranging from -50°C to 0°C, as described later.

The monomers whose homopolymers have a glass transition temperature of greater than or equal to 40°C are chosen, preferably, from the following monomers, also known as the main monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH\text{-}COOR_2$ in which $R_2$ represents a $C_4$ to $C_{12}$ cycloalkyl group such as isobornyl acrylate or a tert-butyl group,
- (meth)acrylamides of formula:

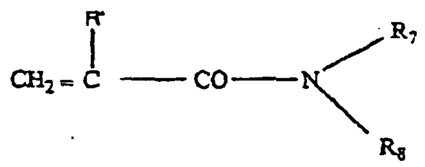

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl. Examples of monomers that may be mentioned include N-butylacrylamide, N-t-butylacrylamide, N-isopropylacrylamide, N,N-dimethylacrylamide and N,N-dibutylacrylamide,

- and mixtures thereof.

Main monomers that are particularly preferred are methyl methacrylate, isobutyl methacrylate and isobornyl (meth)acrylate, and mixtures thereof.

b) Block with a Tg of less than or equal to 20°C

The block with a Tg of less than or equal to 20°C has, for example, a Tg ranging from -100 to 20°C, preferably less than or equal to 15°C, especially ranging from -80°C to 15°C and better still less than or equal to 10°C, for example ranging from -50°C to 0°C.

The block with a Tg of less than or equal to 20°C may be a homopolymer or a copolymer.

The block with a Tg of less than or equal to 20°C may be totally or partially derived from one or more monomers, which are such that the homopolymer prepared from these monomers has a glass transition temperature of less than or equal to 20°C.

In the case where this block is a homopolymer, it is derived from monomers which are such that the homopolymers prepared from these monomers have glass transition temperatures of less than or equal to 20°C. This second block may be a homopolymer consisting of only one type of monomer (for which the Tg of the corresponding homopolymer is less than or equal to 20°C).

In the case where the block with a Tg of less than or equal to 20°C is a copolymer, it may be totally or partially derived from one or more monomers, the nature and concentration of which are chosen such that the Tg of the resulting copolymer is less than or equal to 20°C.

It may comprise, for example

- one or more monomers whose corresponding homopolymer has a Tg of less than or equal to 20°C, for example a Tg ranging from -100°C to 20°C, preferably less than 15°C, especially ranging from -80°C to 15°C and better still less than 10°C, for example ranging from -50°C to 0°C, and
- one or more monomers whose corresponding homopolymer has a Tg of greater than 20°C, such as monomers with a Tg of greater than or equal to 40°C, for example a Tg ranging from 40 to 150°C, preferably greater than or equal to 50°C, for example ranging from 50°C to 120°C and better still greater than or equal to 60°C, for example ranging

from 60°C to 120°C and/or monomers with a Tg of between 20 and 40°C, as described above.

Preferably, the block with a Tg of less than or equal to 20°C is a homopolymer.

The monomers whose homopolymer has a Tg of less than or equal to 20°C are preferably chosen from the following monomers, or main monomer:

- acrylates of formula $CH_2 = CHCOOR_3$, $R_3$ representing a linear or branched $C_1$ to $C_{12}$ unsubstituted alkyl group, with the exception of the tert-butyl group, in which one or more hetero atoms chosen from O, N and S is (are) optionally intercalated,
- methacrylates of formula $CH_2 = C(CH_3)\text{-}COOR_4$, $R_4$ representing a linear or branched $C_6$ to $C_{12}$ unsubstituted alkyl group, in which one or more hetero atoms chosen from O, N and S is (are) optionally intercalated;
- vinyl esters of formula $R_5\text{-CO-O-CH} = CH_2$ in which $R_5$ represents a linear or branched $C_4$ to $C_{12}$ alkyl group,
- $C_4$ to $C_{12}$ alkyl vinyl ethers,
- N-($C_4$ to $C_{12}$)alkyl acrylamides, such as N-octylacrylamide,
- and mixtures thereof.

The main monomers that are particularly preferred for the block with a Tg of less than or equal to 20°C are alkyl acrylates whose alkyl chain contains from 1 to 10 carbon atoms, with the exception of the tert-butyl group, such as methyl acrylate, isobutyl acrylate and 2-ethylhexyl acrylate, and mixtures thereof.

c) Block with a Tg of between 20 and 40°C

The block with a Tg of between 20 and 40°C may be a homopolymer or a copolymer.

The block with a Tg of between 20 and 40°C may be totally or partially derived from one or more monomers, which are such that the homopolymer prepared from these monomers has a glass transition temperature of between 20 and 40°C.

The block with a Tg of between 20 and 40°C may be totally or partially derived from monomers, which are such that the corresponding homopolymer has a Tg of greater than or equal to 40°C and from monomers which are such that the corresponding homopolymer has a Tg of less than or equal to 20°C.

In the case where this block is a homopolymer, it is derived from monomers (or main monomer) which are such that the homopolymers prepared from these monomers have glass transition temperatures of between 20 and 40°C. This first block may be a homopolymer, consisting of only one type of monomer (for which the Tg of the corresponding homopolymer ranges from 20°C to 40°C).

The monomers whose homopolymer has a glass transition temperature of between 20 and 40°C are preferably chosen from n-butyl methacrylate, cyclodecyl acrylate, neopentyl acrylate and isodecylacrylamide, and mixtures thereof.

In the case where the block with a Tg of between 20 and 40°C is a copolymer, it is totally or partially derived from one or more monomers (or main monomer) whose nature and concentration are chosen such that the Tg of the resulting copolymer is between 20 and 40°C.

Advantageously, the block with a Tg of between 20 and 40°C is a copolymer totally or partially derived from:

- main monomers whose corresponding homopolymer has a Tg of greater than or equal to 40°C, for example a Tg ranging from 40°C to 150°C, preferably greater than or equal to 50°C, for example ranging from 50 to 120°C and better still greater than or equal to 60°C, for example ranging from 60°C to 120°C, as described above, and/or
- main monomers whose corresponding homopolymer has a Tg of less than or equal to 20°C, for example a Tg ranging from -100 to 20°C, preferably less than or equal to 15°C, especially ranging from -80°C to 15°C and better still less than or equal to 10°C, for example ranging from -50°C to 0°C, as described above, the said monomers being chosen such that the Tg of the copolymer forming the first block is between 20 and 40°C.

Such main monomers are chosen, for example, from methyl methacrylate, isobornyl acrylate and methacrylate, butyl acrylate and 2-ethylhexyl acrylate, and mixtures thereof.

Preferably, the proportion of the second block with a Tg of less than or equal to 20°C ranges from 10% to 85% by weight, better still from 20% to 70% and even better still from 20% to 50% by weight of the polymer.

Preferably, each of the first and second blocks comprises at least one monomer chosen from acrylic acid, acrylic acid esters, methacrylic acid and methacrylic acid esters, and mixtures thereof.

Advantageously, each of the first and second blocks is totally derived from at least one monomer chosen from acrylic acid, acrylic acid esters, methacrylic acid and methacrylic acid esters, and mixtures thereof.

However, each of the blocks may contain in small proportion at least one constituent monomer of the other block.

Thus, the first block may contain at least one constituent monomer of the second block, and vice versa.

Each of the first and/or second blocks may comprise, in addition to the monomers indicated above, one or more other monomers known as additional monomers, which are different from the main monomers mentioned above.
The nature and amount of this or these additional monomer(s) are chosen such that the block in which they are present has the desired glass transition temperature.
This additional monomer is chosen, for example, from:

a) hydrophilic monomers such as:

- ethylenically unsaturated monomers comprising at least one carboxylic or sulfonic acid function, for instance:

  acrylic acid, methacrylic acid, crotonic acid, maleic anhydride, itaconic acid, fumaric acid, maleic acid, acrylamidopropanesulfonic acid, vinylbenzoic acid, vinylphosphoric acid, and salts thereof,

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof,
- methacrylates of formula $CH_2 = C(CH_3)\text{-}COOR_6$ in which $R_6$ represents a linear or branched alkyl group containing from 1 to 4 carbon atoms, such as a methyl, ethyl, propyl or isobutyl group, the said alkyl group being substituted with one or more substituents chosen from hydroxyl groups (for instance 2-hydroxypropyl methacrylate and 2-hydroxyethyl methacrylate) and halogen atoms (Cl, Br, I or F), such as trifluoroethyl methacrylate,
- methacrylates of formula $CH_2 = C(CH_3)\text{-}COOR_9$, $R_9$ representing a linear or branched $C_6$ to $C_{12}$ alkyl group in which one or more hetero atoms chosen from O, N and S is (are) optionally intercalated, the said alkyl group being substituted with one or more substituents chosen from hydroxyl groups and halogen atoms (Cl, Br, I or F);
- acrylates of formula $CH_2 = CHCOOR_{10}$, $R_{10}$ representing a linear or branched $C_1$ to $C_{12}$ alkyl group substituted with one or more substituents chosen from hydroxyl groups and halogen atoms (Cl, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit 5 to 30 times, for example methoxy-POE, or
  $R_{10}$ represents a polyoxyethylenated group comprising from 5 to 30 ethylene oxide units b) ethylenically unsaturated monomers comprising one or more silicon atoms, such as methacryloxypropyltrimethoxysilane and methacryloxypropyl-tris(trimethylsiloxy)silane,
- and mixtures thereof.

Additional monomers that are particularly preferred are acrylic acid, methacrylic acid and trifluoroethyl methacrylate, and mixtures thereof.
According to one embodiment, each of the first and second blocks of the block polymer comprises at least one monomer chosen from (meth)acrylic acid esters and optionally at least one additional monomer such as (meth)acrylic acid, and mixtures thereof.
According to another embodiment, each of the first and second blocks of the block polymer is totally derived from at least one monomer chosen from (meth)acrylic acid esters and optionally at least one additional monomer such as (meth)acrylic acid, and mixtures thereof.
According to one preferred embodiment, the block polymer is a non-silicone polymer, i.e. a polymer free of silicon atoms.
This or these additional monomer(s) generally represent(s) an amount of less than or equal to 30% by weight, for example from 1% to 30% by weight, preferably from 5% to 20% by weight and more preferably from 7% to 15% by weight, relative to the total weight of the first and/or second blocks.
The block polymer may be obtained by free-radical solution polymerization according to the following preparation process:

- a portion of the polymerization solvent is introduced into a suitable reactor and heated until the adequate temperature for the polymerization is reached (typically between 60 and 120°C),
- once this temperature is reached, the constituent monomers of the first block are introduced in the presence of some of the polymerization initiator,
- after a time T corresponding to a maximum degree of conversion of 90%, the constituent monomers of the second block and the rest of the initiator are introduced,
- the mixture is left to react for a time T' (ranging from 3 to 6 hours), after which the mixture is cooled to room temperature,
- the polymer dissolved in the polymerization solvent is obtained.

First embodiment

According to a first embodiment, the block polymer comprises a first block with a Tg of greater than or equal to 40°C, as described above in a) and a second block with a Tg of less than or equal to 20°C, as described above in b).

Preferably, the first block with a Tg of greater than or equal to 40°C is a copolymer derived from monomers which are such that the homopolymer prepared from these monomers has a glass transition temperature of greater than or equal to 40°C, such as the monomers described above.

Advantageously, the second block with a Tg of less than or equal to 20°C is a homopolymer derived from monomers which are such that the homopolymer prepared from these monomers has a glass transition temperature of less than or equal to 20°C, such as the monomers described above.

Preferably, the proportion of the block with a Tg of greater than or equal to 40°C ranges from 20% to 90%, better still from 30% to 80% and even better still from 50% to 70% by weight of the polymer. Preferably, the proportion of the block with a Tg of less than or equal to 20°C ranges from 5% to 75%, preferably from 15% to 50% and better still from 25% to 45% by weight of the polymer.

Thus, according to a first variant, the polymer according to the invention may comprise:

- a first block with a Tg of greater than or equal to 40°C, for example having a Tg ranging from 70 to 110°C, which is a methyl methacrylate/acrylic acid copolymer,
- a second block with a Tg of less than or equal to 20°C, for example ranging from 0 to 20°C, which is a methyl acrylate homopolymer, and
- an intermediate block which is a methyl methacrylate/acrylic acid/methyl acrylate copolymer.

According to a second variant, the polymer according to the invention may comprise:

- a first block with a Tg of greater than or equal to 40°C, for example ranging from 70 to 100°C, which is a methyl methacrylate/acrylic acid/trifluoroethyl methacrylate copolymer,
- a second block with a Tg of less than or equal to 20°C, for example ranging from 0 to 20°C, which is a methyl acrylate homopolymer, and
- an intermediate block which is a methyl methacrylate/acrylic acid/methyl acrylate/trifluoroethyl methacrylate random copolymer.

According to a third variant, the polymer according to the invention may comprise:

- a first block with a Tg of greater than or equal to 40°C, for example ranging from 85 to 115°C, which is an isobornyl acrylate/isobutyl methacrylate copolymer,
- a second block with a Tg of less than or equal to 20°C, for example ranging from -85 to -55°C, which is a 2-ethylhexyl acrylate homopolymer, and
- an intermediate block, which is an isobornyl acrylate/isobutyl methacrylate/2-ethylhexyl acrylate random copolymer.

According to a fourth variant, the polymer according to the invention may comprise:

- a first block with a Tg of greater than or equal to 40°C, for example ranging from 85 to 115°C, which is an isobornyl acrylate/methyl methacrylate copolymer,
- a second block with a Tg of less than or equal to 20°C, for example ranging from -85 to -55°C, which is a 2-ethylhexyl acrylate homopolymer, and
- an intermediate block which is an isobornyl acrylate/methyl methacrylate/2-ethylhexyl acrylate random copolymer.

According to a fifth variant, the polymer according to the invention may comprise:

- a first block with a Tg of greater than or equal to 40°C, for example ranging from 95 to 125°C, which is an isobornyl acrylate/isobornyl methacrylate copolymer,
- a second block with a Tg of less than or equal to 20°C, for example ranging from -85 to -55°C, which is a 2-ethylhexyl acrylate homopolymer, and
- an intermediate block which is an isobornyl acrylate/isobornyl methacrylate/2-ethylhexyl acrylate random copolymer.

According to a sixth variant, the polymer according to the invention may comprise:

- a first block with a Tg of greater than or equal to 40°C, for example ranging from 85 to 115°C, which is an isobornyl

methacrylate/isobutyl methacrylate copolymer,

- a second block with a Tg of less than or equal to 20°C, for example ranging from -35 to -5°C, which is an isobutyl acrylate homopolymer, and
- an intermediate block which is an isobornyl methacrylate/isobutyl methacrylate/isobutyl acrylate random copolymer.

According to a seventh variant, the polymer according to the invention may comprise:

- a first block with a Tg of greater than or equal to 40°C, for example ranging from 95 to 125°C, which is an isobornyl acrylate/isobornyl methacrylate copolymer,
- a second block with a Tg of less than or equal to 20°C, for example ranging from -35 to -5°C, which is an isobutyl acrylate homopolymer, and
- an intermediate block which is an isobornyl acrylate/isobornyl methacrylate/isobutyl acrylate random copolymer.

According to an eighth variant, the polymer according to the invention may comprise:

- a first block with a Tg of greater than or equal to 40°C, for example ranging from 60 to 90°C, which is an isobornyl acrylate/isobornyl methacrylate copolymer,
- a second block with a Tg of less than or equal to 20°C, for example ranging from -35 to -5°C, which is an isobutyl acrylate homopolymer, and
- an intermediate block which is an isobornyl acrylate/isobutyl methacrylate/isobutyl acrylate random copolymer.

The examples that follow illustrate, in a non-limiting manner, polymers corresponding to this first embodiment.
The amounts are expressed in grams.

**Example 1: Preparation of a poly(methyl methacrylate)/ acrylic acid/methyl acrylate) polymer**

100 g of butyl acetate are introduced into a 1 litre reactor and the temperature is then raised so as to pass from room temperature (25°C) to 90°C in 1 hour. 180 g of methyl methacrylate, 30 g of acrylic acid, 40 g of butyl acetate, 70 g of isopropanol and 1.8 g of 2,5-bis(2-ethylhexanoylperoxy)-2,5-dimethylhexane (Trigonox® 141 from Akzo Nobel) are then added.
The mixture is maintained at 90°C for 1 hour. 90 g of methyl acrylate, 70 g of butyl acetate, 20 g of isopropanol and 1.2 g of 2,5-bis(2-ethylhexanoylperoxy)-2,5-dimethylhexane are then introduced into the above mixture, still at 90°C and over 1 hour.
The mixture is maintained at 90°C for 3 hours and then diluted with 105 g of butyl acetate and 45 g of isopropanol, and the mixture is then cooled.
A solution containing 40% polymer active material in a butyl acetate/isopropanol mixture is obtained.
A polymer comprising a poly(methyl methacrylate/acrylic acid) first block with a Tg of 100°C, a polymethyl acrylate second block with a Tg of 10°C and an intermediate block which is a methyl methacrylate/acrylic acid/polymethyl acrylate random polymer is obtained.
This polymer has a weight-average mass of 52 000 and a number-average mass of 18 000, i.e. a polydispersity index I of 2.89.

**Example 2: Preparation of a poly(isobornyl acrylate/ isobutyl methacrylate/2-ethylhexyl acrylate) polymer**

100 g of isododecane are introduced into a 1 litre reactor and the temperature is then increased so as to pass from room temperature (25°C) to 90°C over 1 hour. 120 g of isobornyl acrylate, 90 g of isobutyl methacrylate, 110 g of isododecane and 1.8 g of 2,5-bis(2-ethylhexanoylperoxy)-2,5-dimethylhexane (Trigonox® 141 from Akzo Nobel) are then added, at 90°C and over 1 hour.
The mixture is maintained at 90°C for 1 hour 30 minutes.
90 g of 2-ethylhexyl acrylate, 90 g of isododecane and 1.2 g of 2,5-bis(2-ethylhexanoyl-peroxy)-2,5-dimethylhexane are then introduced into the above mixture, still at 90°C and over 30 minutes.
The mixture is maintained at 90°C for 3 hours and is then cooled.
A solution containing 50% polymer active material in isododecane is obtained.
A polymer comprising a poly(isobornyl acrylate/isobutyl methacrylate) first block with a Tg of 80°C, a poly-2-ethylhexyl acrylate second block with a Tg of -70°C and an intermediate block which is an isobornyl acrylate/isobutyl methacrylate/2-ethylhexyl acrylate random polymer is obtained.
This polymer has a weight-average mass of 77 000 and a number-average mass of 19 000, i.e. a polydispersity index I of 4.05.

**Example 3: Preparation of a poly(isobornyl acrylate/ isobornyl methacrylate/2-ethylhexyl acrylate) polymer**

100 g of isododecane are introduced into a 1 litre reactor and the temperature is then increased so as to pass from room temperature (25°C) to 90°C over 1 hour. 105 g of isobornyl acrylate, 105 g of isobornyl methacrylate, 110 g of isododecane and 1.8 g of 2,5-bis(2-ethylhexanoylperoxy)-2,5-dimethylhexane (Trigonox® 141 from Akzo Nobel) are then added, at 90°C and over 1 hour.

The mixture is maintained at 90°C for 1 hour 30 minutes.

90 g of 2-ethylhexyl acrylate, 90 g of isododecane and 1.2 g of 2,5-bis(2-ethylhexanoyl-peroxy)-2,5-dimethylhexane are then introduced into the above mixture, still at 90°C and over 30 minutes.

The mixture is maintained at 90°C for 3 hours and is then cooled.

A solution containing 50% polymer active material in isododecane is obtained.

A polymer comprising a poly(isobornyl acrylate/isobornyl methacrylate) first block with a Tg of 110°C, a poly-2-ethylhexyl acrylate second block with a Tg of -70°C and an intermediate block which is an isobornyl acrylate/isobornyl methacrylate/2-ethylhexyl acrylate random polymer is obtained.

This polymer has a weight-average mass of 103 900 and a number-average mass of 21 300, i.e. a polydispersity index I of 4.89.

**Example 4: Preparation of a poly(isobornyl methacrylate/isobutyl methacrylate/isobutyl acrylate) polymer**

100 g of isododecane are introduced into a 1 litre reactor and the temperature is then increased so as to pass from room temperature (25°C) to 90°C over 1 hour. 120 g of isobornyl methacrylate, 90 g of isobutyl methacrylate, 110 g of isododecane and 1.8 g of 2,5-bis(2-ethylhexanoylperoxy)-2,5-dimethylhexane (Trigonox® 141 from Akzo Nobel) are then added, at 90°C and over 1 hour.

The mixture is maintained at 90°C for 1 hour 30 minutes.

90 g of isobutyl acrylate, 90 g of isododecane and 1.2 g of 2,5-bis(2-ethylhexanoyl-peroxy)-2,5-dimethylhexane are then introduced into the above mixture, still at 90°C and over 30 minutes.

The mixture is maintained at 90°C for 3 hours and is then cooled.

A solution containing 50% polymer active material in isododecane is obtained.

A polymer comprising a poly(isobornyl methacrylate/isobutyl methacrylate) first block with a Tg of 95°C, a polyisobutyl acrylate second block with a Tg of -20°C and an intermediate block which is an isobornyl methacrylate/isobutyl methacrylate/isobutyl acrylate random polymer is obtained.

This polymer has a weight-average mass of 100 700 and a number-average mass of 20 800, i.e. a polydispersity index I of 4.85.

**Example 5: Preparation of a poly(isobornyl acrylate/ isobornyl methacrylate/isobutyl acrylate) polymer**

100 g of isododecane are introduced into a 1 litre reactor and the temperature is then increased so as to pass from room temperature (25°C) to 90°C over 1 hour. 105 g of isobornyl acrylate, 105 g of isobornyl methacrylate, 110 g of isododecane and 1.8 g of 2,5-bis(2-ethylhexanoylperoxy)-2,5-dimethylhexane (Trigonox® 141 from Akzo Nobel) are then added, at 90°C and over 1 hour.

The mixture is maintained at 90°C for 1 hour 30 minutes.

90 g of isobutyl acrylate, 90 g of isododecane and 1.2 g of 2,5-bis(2-ethylhexanoyl-peroxy)-2,5-dimethylhexane are then introduced into the above mixture, still at 90°C and over 30 minutes.

The mixture is maintained at 90°C for 3 hours and is then cooled.

A solution containing 50% polymer active material in isododecane is obtained.

A polymer comprising a poly(isobornyl acrylate/isobornyl methacrylate) first block with a Tg of 110°C, a polyisobutyl acrylate second block with a Tg of -20°C and an intermediate block which is an isobornyl acrylate/isobornyl methacrylate/isobutyl acrylate random polymer is obtained.

This polymer has a weight-average mass of 151 000 and a number-average mass of 41 200, i.e. a polydispersity index I of 3.66.

**Example 6: Preparation of a poly(isobornyl acrylate/ isobutyl methacrylate/isobutyl acrylate) polymer**

100 g of isododecane are introduced into a 1 litre reactor and the temperature is then increased so as to pass from room temperature (25°C) to 90°C over 1 hour. 120 g of isobornyl acrylate, 90 g of isobutyl methacrylate, 110 g of isododecane and 1.8 g of 2,5-bis(2-ethylhexanoylperoxy)-2,5-dimethylhexane (Trigonox® 141 from Akzo Nobel) are then added, at 90°C and over 1 hour.

The mixture is maintained at 90°C for 1 hour 30 minutes.

90 g of isobutyl acrylate, 90 g of isododecane and 1.2 g of 2,5-bis(2-ethylhexanoyl-peroxy)-2,5-dimethylhexane are then introduced into the above mixture, still at 90°C and over 30 minutes.

The mixture is maintained at 90°C for 3 hours and is then cooled.

A solution containing 50% polymer active material in isododecane is obtained.

A polymer comprising a poly(isobornyl acrylate/isobutyl methacrylate) first block with a Tg of 75°C, a polyisobutyl acrylate second block with a Tg of -20°C and an intermediate block which is an isobornyl acrylate/isobutyl methacrylate/isobutyl acrylate random polymer is obtained.

This polymer has a weight average mass of 144 200 and a number-average mass of 49 300, i.e. a polydispersity index I of 2.93.

Second embodiment

According to a second embodiment, the block polymer comprises a first block having a glass transition temperature (Tg) of between 20 and 40°C, in accordance with the blocks described in c) and a second block having a glass transition temperature of less than or equal to 20°C, as described above in b) or a glass transition temperature of greater than or equal to 40°C, as described in a) above.

Preferably, the proportion of the first block with a Tg of between 20 and 40°C ranges from 10% to 85%, better still from 30% to 80% and even better still from 50% to 70% by weight of the polymer.

When the second block is a block with a Tg of greater than or equal to 40°C, it is preferably present in a proportion ranging from 10% to 85% by weight, better still from 20% to 70% and even better still from 30% to 70% by weight of the polymer.

When the second block is a block with a Tg of less than or equal to 20°C, it is preferably present in a proportion ranging from 10% to 85% by weight, better still from 20% to 70% and even better still from 20% to 50% by weight of the polymer.

Preferably, the first block with a Tg of between 20 and 40°C is a copolymer derived from monomers which are such that the corresponding homopolymer has a Tg of greater than or equal to 40°C, and from monomers which are such that the corresponding homopolymer has a Tg of less than or equal to 20°C.

Advantageously, the second block with a Tg of less than or equal to 20°C or with a Tg of greater than or equal to 40°C is a homopolymer.

Thus, according to a first variant of this second embodiment, the block polymer may comprise:

- a first block with a Tg of between 20 and 40°C, for example with a Tg of 25 to 39°C, which is a copolymer comprising at least one methyl acrylate monomer, at least one methyl methacrylate monomer and at least one acrylic acid monomer,
- a second block with a Tg of greater than or equal to 40°C, for example ranging from 85 to 125°C, which is a homopolymer composed of methyl methacrylate monomers, and
- an intermediate block comprising at least one methyl acrylate, methyl methacrylate monomer, and
- an intermediate block comprising methyl methacrylate, at least one acrylic acid monomer and at least one methyl acrylate monomer.

According to a second variant of this second embodiment, the block polymer may comprise:

- a first block with a Tg of between 20 and 40°C, for example with a Tg of 21 to 39°C, which is a copolymer comprising isobornyl acrylate/isobutyl methacrylate/2-ethylhexyl acrylate,
- a second block with a Tg of less than or equal to 20°C, for example ranging from -65 to -35°C, which is a methyl methacrylate homopolymer, and
- an intermediate block which is an isobornyl acrylate/isobutyl methacrylate/2-ethylhexyl acrylate random copolymer.

According to a third variant of this second embodiment, the block polymer may comprise:

- a first block with a Tg of between 20 and 40°C, for example with a Tg from 21 to 39°C, which is an isobornyl acrylate/methyl acrylate/acrylic acid copolymer,
- a second block with a Tg of greater than or equal to 40°C, for example ranging from 85 to 115°C, which is an isobornyl acrylate homopolymer, and
- an intermediate block which is an isobornyl acrylate/methyl acrylate/acrylic acid random copolymer.

As a non-limiting illustration, the polymers corresponding to this second embodiment may be prepared as follows.

**Example 7: Preparation of a poly(isobornyl acrylate/ isobutyl methacrylate/2-ethylhexyl acrylate) polymer**

100 g of isododecane are introduced into a 1 litre reactor and the temperature is then increased so as to pass from room temperature (25°C) to 90°C over 1 hour. 54 g of isobornyl acrylate, 75.6 g of isobutyl methacrylate, 50.4 g of 2-ethylhexyl acrylate, 110 g of isododecane and 1.8 g of 2,5-bis(2-ethylhexanoyl-peroxy)-2,5-dimethylhexane (Trigonox® 141 from Akzo Nobel) are then added, at 90°C and over 1 hour. This mixture is maintained at 90°C for 1 hour 30 minutes.

120 g of 2-ethylhexyl acrylate, 90 g of isododecane and 1.2 g of 2,5-bis(2-ethylhexanoyl-peroxy)-2,5-dimethylhexane are then introduced into the above mixture, still at 90°C and over 1 hour.

The mixture is maintained at 90°C for 3 hours and is then diluted and cooled.

A solution containing 50% polymer active material in isododecane is obtained.

The polymer obtained comprises a poly(isobornyl acrylate/isobutyl methacrylate/2-ethylhexyl acrylate) first block with a Tg of 25°C, a poly(2-ethylhexyl acrylate) second block with a Tg of -50°C and an intermediate block which is an isobornyl acrylate/isobutyl methacrylate/2-ethylhexyl acrylate random polymer.

**Example 8: Preparation of a poly(methyl methacrylate/ methyl acrylate/acrylic acid) polymer**

210 g of ethyl acetate are introduced into a 1 litre reactor and the temperature is then increased so as to pass from room temperature (25°C) to 78°C over 1 hour. 54 g of methyl methacrylate, 21 g of acrylic acid, 135 g of methyl acrylate and 1.8 g of 2,5-bis(2-ethylhexanoylperoxy)-2,5-dimethylhexane (Trigonox® 141 from Akzo Nobel) are then added, at 78°C and over 1 hour.

The mixture is maintained at 90°C for 1 hour. 90 g of methyl methacrylate, 90 g of ethyl acetate and 1.2 g of 2,5-bis(2-ethylhexanoylperoxy)-2,5-dimethylhexane are then introduced into the above mixture, still at 78°C and over 1 hour.

The mixture is maintained at 78°C for 3 hours and is then diluted with 150 g of ethyl acetate and cooled.

A solution containing 40% polymer active material in ethyl acetate is obtained.

The polymer obtained comprises a poly(methyl acrylate/methyl methacrylate/acrylic acid) first block with a Tg of 35°C, a poly(methyl methacrylate) second block with a Tg of 100°C and an intermediate block which is a methyl methacrylate/acrylic acid/polymethyl acrylate random polymer.

This polymer has a weight-average mass of 141 000 and a number-average mass of 50 000, i.e. a polydispersity index I of 2.82.

Preferably, the composition according to the invention is a leave-in composition.

Generally, the first composition of the product of the invention contains from 0.1% to 60% by weight of active material (or of solids) of block polymer, preferably from 0.5% to 50% by weight and more preferably from 1% to 40% by weight.

**Second composition**

The cosmetic product according to the invention contains a second composition comprising a second cosmetically acceptable medium.

When the cosmetic product of the invention is a makeup product, the second composition may be any cosmetic makeup composition known to those skilled in the art. In particular, the second composition may be a lipstick, a mascara, a gloss, an eyeshadow, a foundation or a nail varnish.

The second composition is advantageously chosen such that it improves at least one cosmetic property of the first composition, when the first composition is applied alone to the said keratin material. For example, the second composition may improve the comfort of the first composition or reduce its tacky nature.

The second composition may also be chosen such that the product, once applied to the keratin materials, has satisfactory transfer-resistance, gloss and staying-power properties.

In particular, the mean gloss measured at 20° of the first composition, once spread onto a support, is greater than or equal to 30, preferably greater than or equal to 40, preferably greater than or equal to 50 and preferentially greater than or equal to 60 out of 100.

According to one embodiment, the mean gloss of the product measured at 20°, the product being spread onto a support, is greater than or equal to 30, preferably greater than or equal to 40, preferably greater than or equal to 50 and preferentially greater than or equal to 60 out of 100.

**Mean gloss**

The term "mean gloss" means the gloss as may be conventionally measured using a glossmeter by the following

method.

A coat of between 50 μm and 150 μm in thickness of a composition is spread using an automatic spreader onto a Leneta brand contrast card of reference Form 1A Penopac. The composition may be the first or the second composition. The coat covers at least the white background of the card. The deposit is left to dry for 24 hours at a temperature of 30°C, and the gloss at 20° is then measured on the white background using a Byk Gardner brand glossmeter of reference microTri-Gloss.

This measurement (between 0 and 100) is repeated at least three times, and the mean gloss is the mean of the at least three measurements taken.

When it is desired to measure the gloss of the product according to the invention, a first coat of one of the two compositions is spread onto the contrast card, under the same conditions as described previously. This first coat is left to dry, and a second coat of the other composition is then spread on top, under the same conditions as described previously.

The mean gloss of the first composition, or of the second composition, or of the product of the invention, measured at 20° is advantageously greater than or equal to 30, better still greater than or equal to 35, even better still greater than or equal to 40, even better still greater than or equal to 45, even better still greater than or equal to 50 out of 100, even better still greater than or equal to 55, even better still greater than or equal to 60, even better still greater than or equal to 65, even better still greater than or equal to 70 or, even better still, greater than or equal to 75 out of 100. For certain compositions according to the invention, such as nail varnishes, the gloss measured at 20° may be greater than or equal to 70, or even 80 out of 100.

Preferably, the mean gloss of the first composition, or of the second composition, or of the product of the invention, once spread onto a support, measured at 60° is greater than or equal to 50, better still greater than or equal to 60, better still greater than or equal to 65, better still greater than or equal to 70, better still greater than or equal to 75, better still greater than or equal to 80, better still greater than or equal to 85 or, better still, greater than or equal to 90 out of 100.

The measurement of the mean gloss at 60° is performed under the same conditions as those described previously to measure the mean gloss at 20°.

The first composition and/or the second composition may also be chosen such that the product, once applied to the keratin materials, has satisfactory transfer-resistant, gloss and staying-power properties.

In particular, the first composition preferably has a transfer index of less than 40, preferably less than or equal to 30, preferably less than or equal to 20, preferably less than or equal to 15, preferably less than or equal to 10, preferably less than or equal to 5 and more preferably less than or equal to 2 out of 100.

The second composition may be chosen such that the transfer index of the product of the invention is less than or equal to 40, preferably less than or equal to 30, preferably less than or equal to 20, preferably less than or equal to 15, preferably less than or equal to 10 and preferentially less than or equal to 5.

## Transfer index

The transfer index may be measured according to the following method.

A support (rectangle of 40 mm x 70 mm and 3 mm thick) of polyethylene foam that is adhesive on one of the faces, having a density of 33 kg/m$^3$ (sold under the name RE40X70EP3 from the company Joint Technique Lyonnais Ind) is preheated on a hotplate maintained at a temperature of 40°C in order for the surface of the support to be maintained at a temperature of 33°C ± 1°C.

When it is desired to measure the transfer index of the first composition, the first composition is applied over the entire non-adhesive surface of the support, by spreading it using a fine brush to obtain a deposit of about 15 μm of the composition, while leaving the support on the hotplate, and the support is then left to dry for 30 minutes.

When it is desired to measure the transfer index of the product of the invention, the first composition is applied over the entire non-adhesive surface of the support, by spreading it using a fine brush to obtain a deposit of about 15 μm of the composition, while leaving the support on the hotplate, and the support is then dried for 30 minutes. The second composition is then applied.

After drying, the support (coated with the first composition or the product) is bonded via its adhesive face onto an anvil of diameter 20 mm and equipped with a screw pitch. The support/deposit assembly is then cut up using a punch 18 mm in diameter. The anvil is then screwed onto a press (Statif Manuel Imada SV-2 from the company Someco) equipped with a tensile testing machine (Imada DPS-20 from the company Someco).

White photocopier paper of 80 g/m$^2$ is placed on the bed of the press and the support/deposit assembly is then pressed on the paper at a pressure of 2.5 kg for 30 seconds. After removing the support/deposit assembly, some of the deposit is transferred onto the paper. The colour of the deposit transferred onto the paper is then measured using a Minolta CR300 colorimeter, the colour being characterized by the L*, a*, b* colorimetric parameters. The colorimetric parameters $L^*_0$, $a^*_0$ and $b^*_0$ of the colour of the plain paper used is determined.

The difference in colour ΔE1 between the colour of the deposit transferred relative to the colour of the plain paper is then determined by means of the following relationship.

$$\Delta E1 = \sqrt{(L^*-L_0^*)^2+(a^*-a_0^*)^2+(b^*-b_0^*)^2}$$

Moreover, a total transfer reference is prepared by applying the composition or the product directly onto a paper identical to the one used previously, at room temperature (25°C), by spreading the composition using a fine brush and so as to obtain a deposit of about 15 μm of the composition, and the deposit is then left to dry for 30 minutes at room temperature (25°C). After drying, the colorimetric parameters L*', a*' and b*' of the colour of the deposit placed on the paper, corresponding to the reference colour of total transfer, is measured directly. The colorimetric parameters $L^{*'}_0$, $a^{*'}_0$ and $b^{*'}_0$ of the colour of the plain paper used are determined.

The difference in colour ΔE2 between the reference colour of total transfer relative to the colour of the plain paper are then determined by means of the following relationship.

$$\Delta E2 = \sqrt{(L^{*'}-L_0^{*'})^2+(a^{*'}-a_0^{*'})^2+(b^{*'}-b_0^{*'})^2}$$

The transfer of the composition or of the product, expressed as a percentage, is equal to the ratio:

$$100 \times \Delta E1/\Delta E2$$

The measurement is performed on 4 supports in succession and the transfer value corresponds to the mean of the 4 measurements obtained with the 4 supports. The transfer index is equal to the mean of these 4 measurements.

**Organic liquid medium**

The organic medium of the first composition may comprise any cosmetically acceptable, and in general cosmetically acceptable, oil, chosen especially from carbon-based oils, hydrocarbon-based oils, fluoro oils and/or silicone oils of mineral, plant or synthetic origin, alone or as a mixture provided that they form a uniform and macroscopically stable mixture and that they are compatible with the intended use.

The organic medium of the first composition may represent from 5% to 90% of the total weight of the composition and preferably from 20% to 85%. Advantageously, it represents at least 30% of the total weight of the composition.

According to one embodiment, the organic medium comprises one or more volatile oils.

These oils may be hydrocarbon-based oils or silicone oils optionally comprising alkyl or alkoxy groups that are pendent or at the end of a silicone chain.

As volatile silicone oils that may be used in the invention, mention may be made of linear or cyclic silicones with a viscosity at room temperature of less than 8 cSt and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethyl-hexyltrisiloxane, heptamethyloctyltrisiloxane, octamethyltrisiloxane and decamethyltetrasiloxane, and mixtures thereof.

As other volatile oils that may be used in the invention, $C_8$-$C_{16}$ isoalkane oils (also known as isoparaffins), for instance isododecane, isodecane and isohexadecane and, for example, the oils sold under the trade names Isopar and Permethyl, and especially isododecane (Permethyl 99A), are especially preferred.

The volatile oils especially represent from 5% to 85% and better still from 20% to 75% of the total weight of the composition.

According to one embodiment, the fatty phase comprises at least one apolar or sparingly polar non-volatile oil.

As sparingly polar non-volatile oil that may be used in the invention, mention may be made of apolar oils or, especially, oils comprising an alkyl chain especially of $C_3$-$C_{40}$. Examples of apolar or sparingly polar oils that may be mentioned include:

- linear or branched hydrocarbons such as liquid paraffin, liquid petroleum jelly and light naphthas, and hydrogenated

polyisobutene;
- hydrocarbon-based oils of animal origin, for instance squalene;
- fatty alcohols;
- hydrocarbon-based plant oils such as liquid triglycerides of fatty acids of at least 10 carbon atoms;
- synthetic esters and ethers, especially of fatty acids, for instance the oils of formula $R_1C0(O)_xR_2$ in which $R_1$ represents an acid residue containing from 2 to 29 carbon atoms with x being 0 or 1 and $R_2$ represents a hydrocarbon-based chain containing from 3 to 30 carbon atoms, for instance tributyl acetyl citrate, oleyl erucate, 2-octyldodecyl behenate, triisoarachidyl citrate, isocetyl stearoylstearate or octyldodecanyl stearoylstearate, n-propyl acetate, tridecyl trimellitate, diisocetyl dodecanedioleate or stearate, arachidyl propionate, dibutyl phthalate, propylene carbonate or octyldodecyl pentanoate; polyol esters, for instance vitamin F, sorbitan isostearate and glyceryl or diglyceryl triisostearate;
- silicone oils such as polydimethylsiloxanes (PDMS), optionally comprising a $C_3$-$C_{40}$ alkyl or alkoxy chain or a phenylated chain, such as optionally fluorinated phenyltrimethicones and polyalkylmethylsiloxanes, for instance polymethyltrifluoropropyldimethylsiloxanes, or with functional groups such as hydroxyl, thiol and/or amine groups; polysiloxanes modified with fatty acids, fatty alcohols or polyoxyalkylenes,
- fluoro oils;
- mixtures thereof.

These apolar or sparingly polar non-volatile oils may represent from 0.1% to 20% of the total weight of the composition, better still from 0.5% to 10% and even better still from 1% to 5% of the total weight of the composition.
The non-volatile oil is advantageously chosen from hydrocarbons, especially alkanes such as hydrogenated polyisobutene, and from phenylsilicone oils, and mixtures thereof.
The phenylsilicone oils that may be used according to the present invention preferably have a viscosity, measured at 25°C and atmospheric pressure, ranging from 5 to 100 000 cSt and preferably from 5 to 10 000 cSt.
The silicone oil may be, for example, a phenyl trimethicone, a phenyl dimethicone, a phenyl-trimethylsiloxydiphenylsiloxane, a diphenyl dimethicone, a diphenylmethyldiphenyltrisiloxane or a mixture of various phenylsilicone oils, and in particular may correspond to formula (A) below:

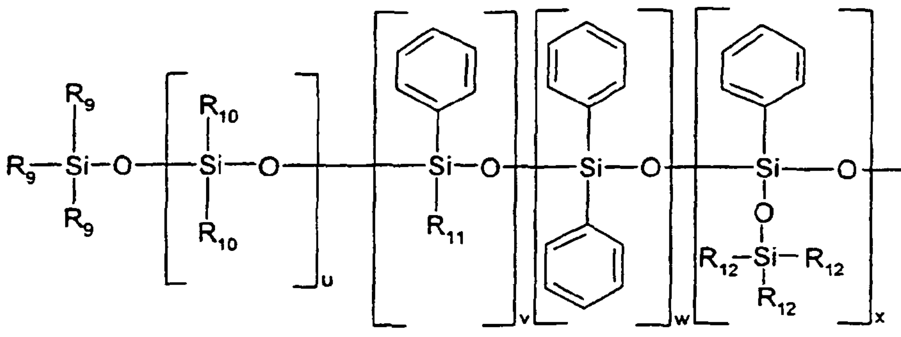

(A)

in which

- $R_9$ and $R_{12}$ are each independently a $C_1$-$C_{30}$ alkyl radical, an aryl radical or an aralkyl radical,
- $R_{10}$ and $R_{11}$ are each independently a $C_1$-$C_{30}$ alkyl radical or an aralkyl radical,
- u, v, w and x are each independently integers ranging from 0 to 900,

with the proviso that the sum v + w + x is other than 0 and that the sum u + v + w + x ranges from 1 to 900; in particular u + v + w + x ranges from 1 to 800.
Advantageously, $R_9$ is a $C_1$-$C_{20}$ alkyl radical, a phenyl radical or an aralkyl radical of the type R'-$C_6H_5$, R' being a $C_1$-$C_5$ alkyl, $R_{10}$ and $R_{11}$ are each independently a $C_1$-$C_{20}$ alkyl radical or an aralkyl radical of the type R'-$C_6H_5$, R' being a $C_1$-$C_5$ alkyl, and $R^{12}$ is a $C_1$-$C_{20}$ alkyl radical.
Preferably, $R_9$ is a methyl, ethyl, propyl, isopropyl, decyl, dodecyl or octadecyl radical, or alternatively a phenyl, tolyl, benzyl or phenethyl radical, $R_{10}$ and $R_{11}$ are each independently a methyl, ethyl, propyl, isopropyl, decyl, dodecyl or octadecyl radical, or alternatively a tolyl, benzyl or phenethyl radical, and $R_{12}$ is a methyl, ethyl, propyl, isopropyl, decyl, dodecyl or octadecyl radical.
Preferably, the phenylsilicone oil is an oil of low viscosity of formula (A) with the sum u + v + w + x ranging from

1 to 150 and better still from 1 to 100, or even from 1 to 50.

In particular, the low-viscosity phenylsilicone oil satisfies formula (III) below:

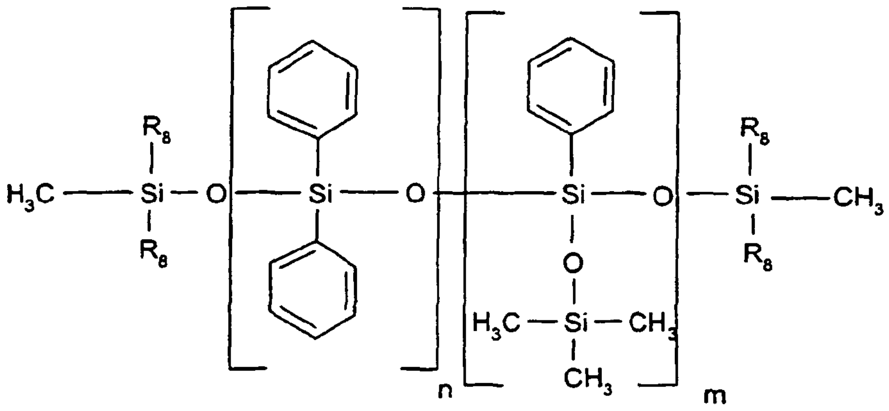

(III)

in which

. $R_8$ is a $C_1$-$C_{30}$ alkyl radical, an aryl radical or an aralkyl radical,
. n is an integer ranging from 0 to 100 and better still less than 100,
. m is an integer ranging from 0 to 100, with the proviso that the sum m + n ranges from 1 to 100 and better still is less than 100.

Advantageously, $R_8$ is a $C_1$-$C_{20}$ alkyl radical, a phenyl radical or an aralkyl radical of the type R'-$C_6H_5$, R' being a $C_1$-$C_5$ alkyl.

Preferably, $R_8$ is a methyl, ethyl, propyl, isopropyl, decyl, dodecyl or octadecyl radical, or alternatively a phenyl, tolyl, benzyl or phenethyl radical. Advantageously, $R_8$ is a methyl radical.

Among the low-viscosity phenylsilicone oils that may be used in the invention, mention may be made of the oils DC556 (22.5 cSt) and SF558 (10-20 cSt) from Dow Corning, the oil Abil AV8853 (4-6 cSt) from Goldschmidt, the oil Silbione 70 633 V 30 (28 cSt) from Rhone-Poulenc, the oils 15 M 40 (50 to 100 cSt) and 15 M 50 (20 to 25 cSt) from PCR, the oils SF 1550 (25 cSt) and PK 20 (20 cSt) from Bayer, the oil Belsil PDM 200 (200 cSt) from Wacker, and the oils KF 53 (175 cSt), KF 54 (400 cSt) and KF 56 (14 cSt) from Shin-Etsu. The values in parentheses represent the viscosities at 25°C.

The organic medium may also contain a polar oil chosen from fatty acid esters of 7 to 29 carbon atoms, for instance diisostearyl malate, isopropyl palmitate, diisopropyl adipate, caprylic/capric acid triglycerides such as those sold by the company Stearineries Dubois or those sold under the names Miglyol 810, 812 and 818 by the company Dynamit Nobel, Shea butter oil, isopropyl myristate, butyl stearate, hexyl laurate, diisopropyl adipate, isononyl isononate, 2-hexyldecyl laurate, 2-octyldecyl palmitate, 2-octyldodecyl myristate or lactate, 2-diethylhexyl succinate, 2-ethylhexyl palmitate, 2-octyldodecyl stearate or castor oil; esters of lanolic acid, of lauric acid or of stearic acid; higher fatty alcohols (of 7 to 29 carbon atoms) such as stearyl alcohol, linoleyl alcohol, linolenyl alcohol, isostearyl alcohol, 2-octyldodecanol, decanol, dodecanol, octadecanol or oleyl alcohol; higher fatty acids (of 7 to 29 carbon atoms) such as myristic acid, palmitic acid, stearic acid, behenic acid, oleic acid, linoleic acid, lino-lenic acid or isostearic acid; mixtures thereof.

These non-volatile polar oils may represent from 0.1% to 10% and better still from 1% to 5% of the total weight of the composition.

When the first or the second composition is intended to be applied to the nails, the cosmetically acceptable medium preferably contains a solvent chosen from:

- ketones that are liquid at room temperature, such as methyl ethyl ketone, methyl isobutyl ketone, diisobutyl ketone, isophorone, cyclohexanone or acetone;
- alcohols that are liquid at room temperature, such as ethanol, isopropanol, diacetone alcohol, 2-butoxyethanol or cyclohexanol;
- glycols that are liquid at room temperature, such as ethylene glycol, propylene glycol, pentylene glycol or glycerol;
- propylene glycol ethers that are liquid at room temperature, such as propylene glycol monomethyl ether, propylene glycol monomethyl ether acetate or dipropylene glycol mono-n-butyl ether;

- cyclic ethers such as γ-butyrolactone;
- short-chain esters (containing from 3 to 8 carbon atoms in total) such as ethyl acetate, methyl acetate, propyl acetate, isopropyl acetate, n-butyl acetate, isopentyl acetate, methoxypropyl acetate or butyl lactate;
- ethers that are liquid at room temperature, such as diethyl ether, dimethyl ether or dichlorodiethyl ether;
- alkanes that are liquid at room temperature, such as decane, heptane, dodecane or cyclohexane;
- alkyl sulfoxides such as dimethyl sulfoxide;
- aldehydes that are liquid at room temperature, such as benzaldehyde or acetaldehyde;
- heterocyclic compounds such as tetrahydrofuran;
- propylene carbonate or ethyl 3-ethoxypropionate;
- mixtures thereof.

Methyl acetate, isopropyl acetate, methoxypropyl acetate, butyl lactate, acetone, methyl ethyl ketone, diacetone alcohol, γ-butyrolactone, tetrahydrofuran, propylene carbonate, ethyl 3-ethoxypropionate and dimethyl sulfoxide, and mixtures thereof, are particularly preferred.

The first composition may comprise, besides the block polymer, an additional polymer such as a film-forming polymer. The second composition may also contain a film-forming polymer. According to the present invention, the term "film-forming polymer" means a polymer capable of forming, by itself or in the presence of an auxiliary film-forming agent, a continuous film that adheres to a support, especially to keratin materials.

Among the film-forming polymers that may be used in the composition of the present invention, mention may be made of synthetic polymers, of free-radical type or of polycondensate type, and polymers of natural origin, and mixtures thereof.

The film-forming polymer may be chosen in particular from cellulose-based polymers such as nitrocellulose, cellulose acetate, cellulose acetobutyrate, cellulose acetopropionate or ethylcellulose, or from polyurethanes, acrylic polymers, vinyl polymers, polyvinyl butyrals, alkyd resins, resins derived from aldehyde condensation products, such as arylsulfonamide-formaldehyde resins, for instance toluenesulfonamide-formaldehyde resin, and arylsulfonamide epoxy resins.

Film-forming polymers that may especially be used include nitrocellulose RS 1/8 sec.; RS ¼ sec.; ½ sec.; RS 5 sec.; RS 15 sec.; RS 35 sec.; RS 75 sec.; RS 150 sec.; AS ¼ sec.; AS ½ sec.; SS ¼ sec.; SS ½ sec.; SS 5 sec., sold especially by the company Hercules; the toluenesulfonamide-formaldehyde resins "Ketjentflex MS80" from the company Akzo or "Santolite MHP" and "Santolite MS80" from the company Faconnier or "Resimpol 80" from the company Pan Americana, the alkyd resin "Beckosol Ode 230-70-E" from the company Dainippon, the acrylic resin "Acryloid B66" from the company Rohm & Haas, and the polyurethane resin "Trixene PR 4127" from the company Baxenden.

The additional film-forming polymer may be present in the composition according to the invention in a content ranging from 0.1% to 60% by weight, preferably ranging from 2% to 40% by weight and better still from 5% to 25% by weight, relative to the total weight of the composition.

The first or the second composition may also comprise at least one plasticizer. The amount of plasticizer may be chosen by a person skilled in the art on the basis of his general knowledge, so as to obtain a composition with cosmetically acceptable properties.

The first composition according to the invention may also comprise, depending on the intended type of application, at least one wax.

For the purposes of the present invention, a wax is a lipophilic fatty compound, that is solid at room temperature (25°C), with a reversible solid/liquid change of state, having a melting point of greater than 40°C which may be up to 200°C, and having an anisotropic crystal organization in the solid state.

Linear hydrocarbon-based waxes are preferred in the context of the present invention. Their melting point is advantageously greater than 35°C, for example greater than 55°C and preferably greater than 80°C.

The linear hydrocarbon-based waxes are advantageously chosen from substituted linear alkanes, unsubstituted linear alkanes, unsubstituted linear alkenes and substituted linear alkenes, an unsubstituted compound being a compound of carbon and hydrogen exclusively. The substituents mentioned above do not contain any carbon atoms.

The linear hydrocarbon-based waxes include ethylene polymers and copolymers with a molecular weight of between 400 and 800, for example Polywax 500 or Polywax 400 sold by New Phase Technologies.

The linear hydrocarbon-based waxes include linear paraffin waxes, for instance the paraffins S&P 206, S&P 173 and S&P 434 from Strahl & Pitsch.

The linear hydrocarbon-based waxes include long-chain linear alcohols, for instance products comprising a mixture of polyethylene and of alcohols containing 20 to 50 carbon atoms, for instance Performacol 425 or Performacol 550 (mixture in 20/80 proportions) sold by New Phase Technologies.

The waxes may be present in a proportion of 0.5-30%, better still from 5% to 20% and preferably between 5% and 15% by weight in the first composition, so as not to excessively reduce the gloss of the composition and of the film applied to the lips and/or the skin.

The cosmetically acceptable medium of the second composition preferably comprises a liquid phase that is non-volatile at room temperature and atmospheric pressure.

The term "non-volatile liquid phase" means any medium capable of remaining on the skin or the lips for several hours. A non-volatile liquid phase in particular has a non-zero vapour pressure at room temperature and atmospheric pressure, of less than 0.02 mmHg (2.66 Pa) and better still less than $10^{-3}$ mmHg (0.13 Pa).

The non-volatile liquid phase of the second composition may be at least one hydrocarbon-based oil, a liquid silicone phase, a fluoro phase that is liquid at room temperature, or a mixture thereof.

The term "hydrocarbon-based oil" means oils mainly containing carbon atoms and hydrogen atoms, and in particular alkyl or alkenyl chains, for instance alkanes or alkenes, but also oils containing an alkyl or alkenyl chain comprising one or more ether, ester or carboxylic acid groups.

The non-volatile liquid phase of the second composition advantageously represents 1% to 100%, preferably from 5% to 95%, better still from 20% to 80% and even better still from 40% to 80% of the total weight of the second composition.

The non-volatile liquid phase of the second composition advantageously contains a silicone oil, for example a phenylsilicone oil or a polydimethylsiloxane oil.

The non-volatile silicone oil may be chosen from polyalkylsiloxanes, polyarylsiloxanes and polyalkylarylsiloxanes, and mixtures thereof.

The non-volatile silicone oil may be chosen from:

- linear or branched non-volatile polydimethylsiloxanes (PDMS);
- polydimethylsiloxanes comprising alkyl, alkoxy or phenyl groups, which are pendent or at the end of a silicone chain, these groups containing from 2 to 24 carbon atoms;
- phenylsilicones, for instance phenyl trimethicones, phenyl dimethicones, phenyltrimethylsiloxydiphenyl-siloxanes, diphenyl dimethicones, diphenylmethyl-diphenyltrisiloxanes and 2-phenylethyl trimethyl-siloxysilicates.

The polyalkylsiloxanes according to the invention include polydimethylsiloxanes, (polydimethylsiloxane) (methylvinylsiloxane) copolymers, poly-(dimethylsiloxane)(diphenyl)siloxanes, poly(dimethylsiloxane)(diphenyl)(methylvinylsiloxane) copolymers, and mixtures thereof.

The non-volatile silicone oil that is preferred is an oil chosen from the silicones of formula (II):

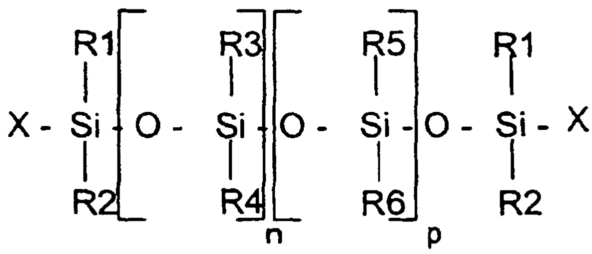

(II)

in which:

$R_1$, $R_2$, $R_5$ and $R_6$ are, together or separately, an alkyl radical containing 1 to 6 carbon atoms,
$R_3$ and $R_4$ are, together or separately, an alkyl radical containing from 1 to 6 carbon atoms or an aryl radical, X is an alkyl radical containing from 1 to 6 carbon atoms, a hydroxyl radical or a vinyl radical,
n and p being chosen so as to give the oil a weight-average molecular mass of less than 200 000 g/mol, preferably less than 150 000 g/mol and more preferably less than 100 000 g/mol.

A polydimethylsiloxane with a viscosity of between 0.5 and 60 000 cSt, preferably between 0.5 and 10 000 cSt and preferentially between 0.5 and 1000 cSt measured according to ASTM standard D-445, for example DC 200 of viscosity 350 cSt sold by Dow Corning, is chosen, for example, as non-volatile silicone of formula (II).

According to one embodiment, the second composition contains a non-volatile silicone of formula (II) such as a polydimethylsiloxane with a viscosity preferably of between 0.5 and 500 cSt and more preferably between 1 and 10 cSt, for example the polydimethylsiloxane sold under the name DC 200 of viscosity 5 cSt and of molecular weight 800, sold by Dow Corning.

The weight-average molecular mass of the silicone oil is preferably between 400 and 200 000, preferably between 4000 and 100 000, preferably between 4000 and 20 000, preferably between 400 and 2000 and more preferably between 400 and 1000 g/mol.

According to one embodiment, the second composition advantageously contains a mixture of a polydimethylsiloxane with a molecular mass of between 200 000 and 300 000 g/mol and a polydimethylsiloxane with a molecular mass of between 400 and 1000 g/mol.

The mass proportion of the silicone compound of high molecular weight/liquid silicone compound is preferably between 20/80 and 60/40 and preferentially between 35/65 and 45/55.

Preferably, the non-volatile liquid phase of the second composition comprises at least one fluoro oil of formula (III):

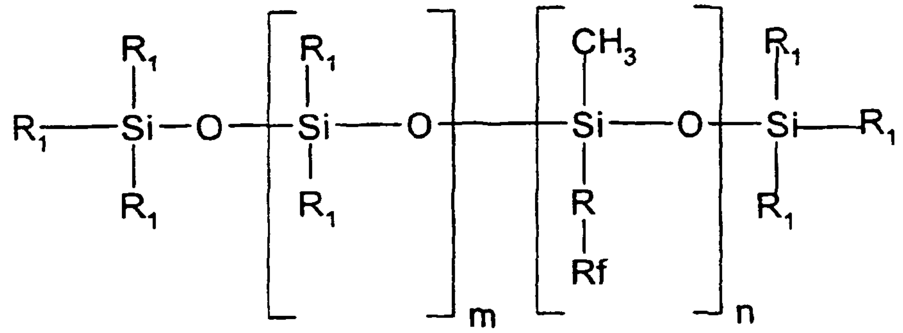

(III)

in which:

- R represents a linear or branched divalent alkyl group containing 1 to 6 carbon atoms, preferably a divalent methyl, ethyl, propyl or butyl group,
- Rf represents a fluoroalkyl radical, especially a perfluoroalkyl radical, containing 1 to 9 carbon atoms, preferably 1 to 4 carbon atoms,
- $R_1$ represent, independently of each other, a $C_1$-$C_{20}$, preferably $C_1$-$C_4$ alkyl radical, a hydroxyl radical or a phenyl radical,
- m is chosen from 0 to 150 and preferably from 20 to 100, and
- n is chosen from 1 to 300 and preferably from 1 to 100.

The oils of formula (III) such that R1 is a methyl, R is an ethyl and Rf is CF3 are preferred. Fluorosilicone compounds of formula (III) that may especially be mentioned are those sold by the company Shin Etsu under the names "X22-819", "X22-820", "X22-821" and "X22-822" or "FL-100".

Among the fluoro oils that may also be mentioned are the fluoro polyethers chosen from the compounds of formula (IV) below:

$R_6$-($CF_2$-$CFR_3$-$CF_2$O)p-($CFR_4$-$CF_2$-O)q-($CFR_5$-O)r-R-, (IV) in which:

- $R_3$ to $R_6$ represent, independently of each other, a monovalent radical chosen from -F, -($CF_2$)n-$CF_3$ and
- O-($CF_2$)n-$CF_3$,
- $R_7$ represents a monovalent radical chosen from -F and
- ($CF_2$)n-$CF_3$,
- with n ranging from 0 to 4 inclusive,
- p ranging from 0 to 600, q ranging from 0 to 860, r ranging from 0 to 1500, and p, q and r being integers chosen such that the weight-average molecular mass of the compound ranges from 500 to 100 000 and preferably from 500 to 10 000.

The fluoro oils may also be chosen from fluoroalkanes chosen from $C_2$-$C_{50}$ and preferably $C_5$-$C_{30}$ perfluoroalkanes and fluoroalkanes, such as perfluoro-decalin, perfluoroadamantane and bromoperfluorooctyl, and mixtures thereof.

The second composition may optionally contain a volatile oil.

These oils may be hydrocarbon-based oils or silicone oils optionally comprising alkyl or alkoxy groups that are pendent or at the end of a silicone chain, or a mixture of these oils.

Preferably, the volatile oils are cosmetic oils chosen from oils with a flash point ranging from 40°C to 100°C, and mixtures thereof. In addition, they advantageously have a boiling point at atmospheric pressure of less than 220°C and better still less than 210°C, especially ranging from 110 to 210°C. Preferably, these volatile oils are not monoalcohols containing at least 7 carbon atoms.

As volatile oils that may be used in the invention, mention may be made of linear or cyclic silicone oils with a viscosity at room temperature of less than 8 cSt and especially containing from 2 to 7 silicon atoms, these silicones optionally comprising alkyl or alkoxy groups of 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltri-siloxane, heptamethyloctyltrisiloxane, hexamethyldisiloxane, octamethyltrisiloxane, decamethyltetrasiloxane and dodecamethylpentasiloxane, and mixtures thereof.

As other volatile oils that may be used in the invention, mention may be made of hydrocarbon-based volatile oils containing from 8 to 16 carbon atoms and mixtures thereof, and especially $C_8$-$C_{16}$ branched alkanes, for instance $C_8$-$C_{16}$ isoalkanes (also known as isoparaffins), isododecane, isodecane and isohexadecane, and for example the oils sold under the trade names Isopar or Permethyl, and $C_8$-$C_{16}$ branched esters, for instance isohexyl neopentanoate, and mixtures thereof.

Isododecane (Permethyl 99 A), $C_8$-$C_{16}$ isoparaffins, for instance Isopar L, E, G or H, and mixtures thereof, optionally combined with decamethyltetrasiloxane or with cyclopentasiloxane, are preferably used.

In general, the amount of volatile oil is used in an amount that is sufficient to improve the spreading qualities of the second composition. This amount will be adapted by a person skilled in the art as a function of the intensity of the desired properties.

The amount of volatile oil is chosen so that it does not reduce the gloss of the second composition. According to one embodiment, the second composition contains no volatile oil.

The second composition advantageously contains a high molecular weight polymer that is different from the non-volatile silicone oil described above.

When the second composition according to the invention is liquid, it advantageously comprises 20% to 50% by weight of a high molecular weight polymer.

When the second composition according to the invention is solid, it advantageously comprises 2% to 40% by weight of a high molecular weight polymer.

The polymer is preferably a silicone polymer.

This polymer may be liquid or solid at room temperature and its weight-average molecular mass is greater than or equal to 200 000 g/mol, preferably between 200 000 and 2 500 000 and preferentially between 200 000 and 2 000 000 g/mol.

The viscosity of this polymer may be between 10 000 and 5 000 000 cSt, preferably between 100 000 and 1 000 000 cSt and more preferably between 300 000 and 700 000 cSt, measured according to ASTM standard D-445.

The high molecular weight polymer is advantageously an ungrafted polymer, i.e. a polymer obtained by polymerizing at least one monomer, without subsequent reaction of the side chains with another chemical compound. The polymer is preferably chosen from dimethiconols, fluorosilicones, dimethicones and mixtures thereof. The polymer is preferably a homopolymer.

In particular, the polymer that may be used is a high molecular weight polymer corresponding to formula (V):

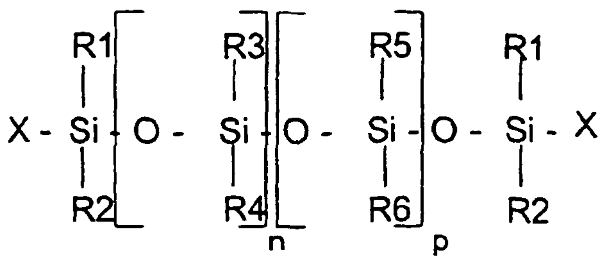

(V)

in which:

$R_1$, $R_2$, $R_5$ and $R_6$ are, together or separately, an alkyl radical containing 1 to 6 carbon atoms, optionally substituted with at least one fluorine atom,

$R_3$ and $R_4$ are, together or separately, an alkyl radical containing from 1 to 6 carbon atoms or an aryl radical, X is

an alkyl radical containing from 1 to 6 carbon atoms, a hydroxyl radical, a vinyl or allyl radical or an alkoxy radical containing from 1 to 6 carbon atoms, n and p being chosen such that the silicone compound has a weight-average molecular mass of greater than or equal to 200 000 g/mol.

p is preferably equal to 0.

The polymers of formula (V) such that $R_1$ to $R_6$ represent a methyl group and the substituent X represents a hydroxyl group are dimethiconols. Examples that are mentioned include the polymers of formula (V) such that p = 0 and n is between 2000 and 40 000 and preferably between 3000 and 30 000. Mention may also be made of polymers with a molecular mass of between 1 500 000 and 2 000 000 g/mol.

According to one embodiment, the high molecular weight polymer is the dimethiconol sold by Dow Corning in a polydimethylsiloxane (5 cSt) under the reference D2-9085, the viscosity of the mixture being equal to 1550 cSt, or the dimethiconol sold by Dow Corning in a polydimethylsiloxane (5 cSt) under the reference DC 1503. The dimethiconol (of molecular weight equal to 1 770 000 g/mol) sold by Dow Corning under the reference Q2-1403 or Q2-1401, the viscosity of the mixture being equal to 4000 cSt, is also preferred.

As high molecular weight polymers that may be used according to the invention, mention may be made of those for which:

- the substituents $R_1$ to $R_6$ and X represent a methyl group, for instance the product sold under the name SE30 by the company General Electric, and the product sold under the name AK 500 000 by the company Wacker,
- the substituents $R_1$ to $R_6$ and X represent a methyl group and p and n are such that the molecular weight is 250 000 g/mol, for instance the product sold under the name Silbione 70047 V by the company Rhodia,
- the substituents $R_1$ to $R_6$ represent a methyl group and the substituent X represents a hydroxyl group, for instance the products sold under the name Q2-1401 or Q2-1403 by the company Dow Corning,
- the substituents $R_1$, $R_2$, $R_5$, $R_6$ and X represent a methyl group, the substituents $R_3$ and $R_4$ represent an aryl group and n and p are such that the molecular weight of the polymer is 600 000 g/mol, for instance the product sold under the name 761 by the company Rhone-Poulenc.

The high molecular weight silicone polymer is preferably introduced into the composition in the form of a mixture with a liquid silicone, the viscosity of the said liquid silicone being between 0.5 and 10 000 cSt, preferably between 0.5 and 500 cSt and more preferably between 1 and 10 cSt.

The fluid silicone may be chosen from polyalkylsiloxanes, polyarylsiloxanes, polyalkylarylsiloxanes and mixtures thereof. The liquid silicone may be a volatile silicone such as a cyclic polydimethylsiloxane comprising 3 to 7 $-(CH_3)_2SiO-$ units.

The liquid silicone may also be a non-volatile polydimethylsiloxane silicone, especially with a viscosity of between 0.5 and 10 000 cSt and more preferably of the order of 5 cSt, for example the silicone sold under the reference DC 200 by Dow Corning.

The proportion of the high molecular weight silicone polymer in the high molecular weight silicone polymer/liquid silicone mixture is preferably between 10/90 and 20/80. The viscosity of the high molecular weight silicone polymer/liquid silicone mixture is preferably between 1000 and 10 000 cSt.

The high molecular weight dimethicones according to the invention include the dimethicones described in patent US 4 152 416. They are sold, for example, under the references SE30, SE33, SE54 and SE76.

The dimethicones according to the invention are, for example, compounds of formula (III) such that $R_1$ to $R_6$ and X are methyls and p = 0. The molecular weight of these polymers is preferably between 200 000 and 300 000 and preferentially between 240 000 and 260 000 g/mol.

The dimethicones according to the invention include polydimethylsiloxanes, (polydimethylsiloxane) (methylvinylsiloxane) copolymers and poly(dimethylsiloxane)(diphenyl)(methylvinylsiloxane) copolymers, and mixtures thereof.

The high molecular weight fluorosilicones according to the invention preferably have a molecular weight of between 200 000 and 300 000 and preferentially between 240 000 and 260 000 g/mol.

According to one of the aspects of the invention, the second composition according to the invention comprises at least one apolar or sparingly polar compound, which may be chosen from oils, gums and/or waxes. The second composition preferably comprises more than 70%, preferably more than 80% by weight and preferentially 100% by weight of apolar or sparingly polar compounds. These apolar or sparingly polar compounds comprise colouring agents or gelling agents.

According to one embodiment, the second composition comprises a mixture of a polymer with a weight-average molecular mass of greater than or equal to 200 000 g/mol and a silicone oil as described above.

According to one embodiment, the second composition is transparent.

The term "transparent composition" means a transparent to translucent composition, i.e. a composition which is such that it transmits at least 40% of light and preferably at least 50% of light with a wavelength of 750 nm.

The transmission is measured using a Cary 300 Scan UV-visible spectrophotometer from the company Varian, according to the following protocol:

The composition is poured above its melting point into a spectrophotometer cuvette of square cross section with a side length of 10 mm.

The sample of the composition is then cooled for 24 hours at 35°C, and then kept in a chamber thermostatically maintained at 20°C for 24 hours.

The light transmitted through the sample of the composition is then measured by spectrophotometer by scanning wavelengths ranging from 700 nm to 800 nm, the measurement being performed in transmission mode.

The percentage of light transmitted through the sample of the composition at a wavelength of 750 nm is then determined.

When the second composition is transparent, it advantageously contains less than 5%, preferably less than 2% and more preferably less than 1% of pigments.

The high molecular weight polymer/liquid silicone compound mixture preferably represents more than 70%, preferably more than 80% by weight, preferably more than 90% by weight and more preferably more than 95% by weight of the second composition.

The second composition may comprise other compounds, which are preferably apolar or sparingly polar. These apolar or sparingly polar compounds are preferably silicone compounds, colouring agents or gelling agents.

According to one embodiment, the second composition comprises only apolar or sparingly polar ingredients.

The second composition advantageously contains at least one wax, especially when it is in solid form.

The waxes may be present in a proportion of 0.5-30% by weight in the composition and better still from 5% to 20% and preferably between 5% and 15% of the composition.

Linear hydrocarbon-based waxes are preferred in the context of the present invention. Their melting point is advantageously greater than 35°C, for example greater than 55°C and preferably greater than 80°C.

The linear hydrocarbon-based waxes are advantageously chosen from substituted linear alkanes, unsubstituted linear alkanes, unsubstituted linear alkenes and substituted linear alkenes, an unsubstituted compound being composed exclusively of carbon and hydrogen. The substituents mentioned above do not contain carbon atoms.

The linear hydrocarbon-based waxes include ethylene polymers and copolymers with a molecular weight of between 400 and 800, for example Polywax 500 or Polywax 400 sold by New Phase Technologies.

The linear hydrocarbon-based waxes include linear paraffin waxes, for instance the paraffins S&P 206, S&P 173 and S&P 434 from Strahl & Pitsch.

The linear hydrocarbon-based waxes include long-chain linear alcohols, for instance products comprising a mixture of polyethylene and of alcohols containing 20 to 50 carbon atoms, especially Performacol 425 or Performacol 550 (mixture in 20/80 proportions) sold by New Phase Technologies.

The second composition advantageously contains a silicone wax, such as a dimethicone comprising alkyl groups at the end of a chain. These alkyl groups preferably contain more than 18 carbon atoms, preferably between 20 and 50 and preferentially between 30 and 45 carbon atoms.

The silicone wax for example corresponds to formula (VI) or (VII)

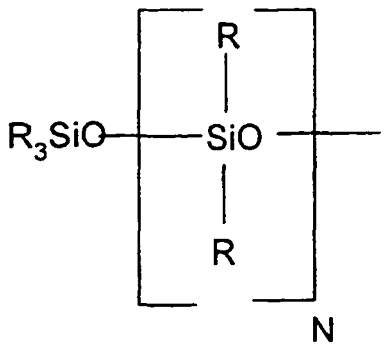

(VI)

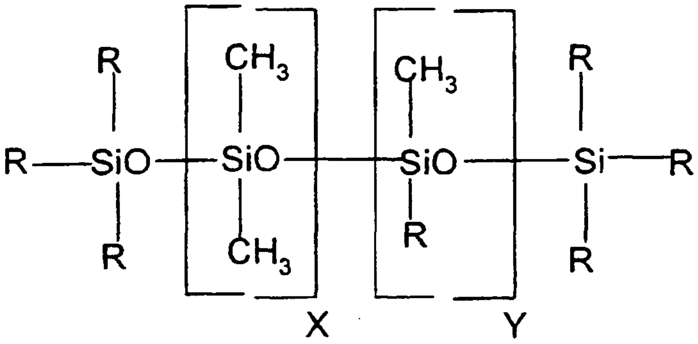

(VII)

in which R is an alkyl, X is greater than or equal to zero and N and Y are greater than or equal to one.

R contains from 1 to 50 carbon atoms provided that the compound is solid at room temperature.

Examples of silicone waxes include:

- $C_{20-24}$ alkyl methicone, $C_{24-28}$ alkyl dimethicone, C2o-24 alkyl dimethicone and $C_{24-28}$ alkyl dimethicone sold by Archimica Fine Chemicals under the reference Silcare 41M40, SilCare 41M50, SilCare 41M70 and SilCare 41M80,
- the stearyl dimethicones of reference SilCare 41M65 sold by Archimica or of reference DC-2503 sold by Dow Corning,
- the stearoxytrimethylsilanes sold under the reference SilCare 1M71 or DC-580,
- the products Abil Wax 9810, 9800 or 2440 from Wacker-Chemie GmbH,
- the $C_{30-45}$ alkyl methicones sold by Dow Corning under the reference AMS-C30 Wax, and also the $C_{30-45}$ alkyl-dimethicones sold by General Electric under the reference SF 1642 or SF 1632.

The first and/or the second composition of the cosmetic product according to the invention may contain a colouring agent that may be chosen from water-soluble or liposoluble dyes, pigments and nacres, and mixtures thereof.

The term "pigments" should be understood as meaning white or coloured, mineral or organic particles, which are insoluble in the liquid organic phase and which are intended to colour and/or opacify the first composition.

The term "nacres" should be understood as meaning iridescent particles produced especially by certain molluscs in their shell, or else synthesized, which are insoluble in the medium of the first composition.

The term "dyes" should be understood as meaning generally organic compounds that are soluble in fatty substances, for instance oils, or soluble in an aqueous-alcoholic phase.

The liposoluble dyes are, for example, Sudan Red, D&C Red No. 17, D&C Green No. 6, β-carotene, soybean oil, Sudan Brown, D&C Yellow No. 11, D&C Violet No. 2, D&C Orange No. 5, quinoline yellow, annatto and bromo acids.

The water-soluble dyes are, for example, beetroot juice, methylene blue and caramel.

The pigments may be interference or non-interference, mineral and/or organic, white or coloured pigments. Among the mineral pigments that may be mentioned are titanium dioxide, optionally surface-treated, zirconium oxide or cerium oxide, and also zinc oxide, iron oxide (black, yellow, brown or red) or chromium oxide, manganese violet, ultramarine blue, chromium hydrate and ferric blue. Among the organic pigments that may be mentioned are carbon black, pigments of barium, strontium, calcium or aluminium organic lake type, including those submitted for certification by the Food and Drug Administration (FDA) (for example D&C or FD&C) and those exempt from FDA certification, for instance lakes based on cochineal carmine.

The nacres or nacreous pigments may be chosen from white nacreous pigments such as mica coated with titanium or with bismuth oxychloride, coloured nacreous pigments such as titanium mica with iron oxides, titanium mica with, especially, ferric blue or chromium oxide, titanium mica with an organic pigment of the abovementioned type, and also nacreous pigments based on bismuth oxychloride. Pigments with goniochromatic properties and/or pigments with a metallic effect as described in the patent application filed under the number FR 0209246, the content of which is incorporated by reference into the present patent application, may thus be used.

In general, the colouring agents represent from 0.001% to 60%, better still from 0.01% to 50% and even better still from 0.1% to 40% of the total weight of each first and second composition.

The first and/or the second composition of the invention may also contain one or more cosmetic or dermatological active agents such as those conventionally used.

As cosmetic, dermatological, hygiene or pharmaceutical active agents that may be used in the composition of the invention, mention may be made of moisturizers, vitamins, essential fatty acids, sphingo-lipids and sunscreens. These active agents are used in the usual amount for those skilled in the art and especially at concentrations of from 0

to 20% and in particular from 0.001% to 15% relative to the total weight of the first or second composition.

The composition may also comprise any other additive usually used in such compositions, such as water, antioxidants, fragrances, preserving agents and essential oils.

Needless to say, a person skilled in the art will take care to select this or these optional compounds, and/or the amount thereof, such that the advantageous properties of the composition are not, or are not substantially, adversely affected by the envisaged addition.

The compositions of the product may be in the form of a cast product, for example in the form of a stick or wand, or in the form of a dish that may be used by direct contact or with a sponge. In particular, they find an application as cast foundations, cast makeup rouges or eyeshadows, lipsticks, lipcare bases or lipcare balms, concealer products and nail varnishes. They may also be in the form of a soft paste or a gel, a more or less fluid cream, or a liquid packaged in a tube.

The compositions of the product according to the invention may especially constitute a cosmetic care composition for the face, the neck, the hands or the body (for example a care cream, an antisun oil or a body gel), a makeup composition (for example a makeup gel, a cream or a stick) or a composition for artificially tanning or protecting the skin.

The compositions of the product according to the invention may be in the form of a care composition for the skin and/or the integuments or in the form of an antisun composition or a body hygiene composition, especially in the form of a deodorant. They are then especially in uncoloured form. They may then be used as a care base for the skin, the integuments or the lips (lip balms, for protecting the lips against the cold and/or sunlight and/or the wind, or care creams for the skin, the nails or the hair).

For the purposes of the invention, the term "cosmetically acceptable" means a composition of pleasant appearance, odour and feel.

Each composition of the product according to the invention may be in any galenical form normally used for topical application and especially in the form of an oily or aqueous solution, an oily or aqueous gel, an oil-in-water or water-in-oil emulsion, a multiple emulsion, a dispersion of oil in water using vesicles, the vesicles being located at the oil/water interface, or a powder. Each composition may be fluid or solid.

According to one embodiment, the first or the second composition, or both, have a continuous fatty phase preferably in anhydrous form and may contain less than 5% water and better still less than 1% water relative to the total weight of the first or second composition.

Each first and second composition may have the appearance of a lotion, a cream, an ointment, a soft paste, a salve, a solid that has been cast or moulded, especially as a stick or a dish, or a compacted solid.

Each composition may be packaged separately in the same packaging article, for example in a two-compartment pen, the base composition being delivered from one end of the pen and the top composition being delivered from the other end of the pen, each end being especially closed in a leaktight manner with a lid.

Alternatively, each of the compositions may be packaged in a different packaging article.

Preferably, the composition that is applied as the first coat is in liquid or pasty form, which is highly desirable in the case of a lipstick or an eyeliner.

The product according to the invention may be advantageously used for making up the skin and/or the lips and/or the integuments depending on the nature of the ingredients used. In particular, the product of the invention may be in the form of a solid foundation, a lipstick wand or paste, a concealer product, an eye contour product, an eyeliner, a mascara, an eyeshadow, a body-makeup product or a skin-colouring product.

Advantageously, the second composition has care, gloss or transparency properties.

A subject of the invention is also a lip product, a varnish, a mascara, a foundation, a tattoo, a makeup rouge or an eyeshadow comprising a first and a second composition as described above.

The compositions of the product of the invention may be obtained by heating the various constituents to the melting point of the highest-melting waxes, followed by casting the molten mixture in a mould (dish or finger stall). They may also be obtained by extrusion, as described in patent application EP-A-0 667 146.

The invention is illustrated in greater detail in the following examples. The percentages are percentages by weight.

**Examples 9 to 13: First composition**

| **Example** | **9** | **10** | **11** | **12** | **13** |
|---|---|---|---|---|---|
| Polymer of Example 2 | 90.7 | | | | |
| Polymer of Example 3 | | 90.7 | | | |
| Polymer of Example 6 | | | 90.7 | | |

(continued)

| Example | 9 | 10 | 11 | 12 | 13 |
|---|---|---|---|---|---|
| Polymer of Example 5 | | | | 90.7 | |
| Polymer of Example 4 | | | | | 90.7 |
| Hydrogenated polyisobutene | 2.1 | 2.1 | 2.1 | 2.1 | 2.1 |
| Octyldodecanol | 0.9 | 0.9 | 0.9 | 0.9 | 0.9 |
| Phenyl trimethicone (DC 556, 20 cSt, Dow Corning) | 2.1 | 2.1 | 2.1 | 2.1 | 2.1 |
| Copolymer vinylpyrrolidone/1-eicosene (Antaron V-220, ISP) | 1.2 | 1.2 | 1.2 | 1.2 | 1.2 |
| Pigments | 3 | 3 | 3 | 3 | 3 |

<u>Procedure</u>

1. A ground pigmentary mixture of the pigments in the oily phase is prepared by treating the mixture three times in a three-roll mill.
2. The ground material required for the composition and the other ingredients are weighed out in a beaker.
3. The mixture is stirred using a Rayneri blender for 45 minutes.
4. The formula is cast in isododecane-leaktight cooling boxes.

<u>Gloss measurement</u>

1. Films with a wet thickness of 50 and/or 150 µm are prepared using a mechanical applicator. The depositions are made on a contrast card.
2. The films are left to dry for 24 hours at a regulated temperature of 30°C.
3. The gloss measurements are performed using a Byk Gardner micro-tri-gloss glossmeter with measuring angles of 20° and 60°.

The gloss results obtained are given in the table below:

Wet 150 µm deposit

| Angle | Deposit | Example | Mean (%) | Standard deviation |
|---|---|---|---|---|
| Angle of 20° | 150 µm deposit | Example 9 | 54.2 | 2.0 |
| | | Example 10 | 41.0 | 3.5 |
| Ang le | 150 µm dep | Example 9 | 75.7 | 0.8 |

| | | | | |
|---|---|---|---|---|
| | | Example 10 | 73.6 | 1.6 |

Wet 50 µm deposit (closer when dry to the thickness applied to the lips)

| Angle | Deposit | Example | Mean (%) | Standard deviation |
|---|---|---|---|---|
| Angle of 20° | 50 µm deposit | Example 9 | 47.6 | 1.2 |
| | | Example 10 | 42.6 | 5.2 |
| Angle of 60° | 50 µm deposit | Example 9 | 69.3 | 0.7 |
| | | Example 10 | 74.8 | 1.0 |

**Example 14: Second liquid composition**

| | |
|---|---|
| Polydimethylsiloxane sold under the reference Silbione 70047 V by Rhodia (500 000 cSt - 250 000 g/mol) | 40% |
| Polydimethylsiloxane sold by the company Dow Corning under the reference DC200 (5 cSt) | 60% |

The two ingredients are mixed together at 70°C using a Rayneri blender.

**Example 15: Second solid composition**

| | % by weight |
|---|---|
| Silicone oil (PDMS) | 25 |
| DC200 from Dow Corning (5 cSt) | |
| Dimethicone (and) Dimethiconol | 61 |
| D2-9085 from Dow Corning (1550 cSt) | |
| Trifluoropropyl dimethicone (100 cSt) | 1 |
| X22-819 from Shin-Etsu | |
| C30-45 Alkyl dimethicone (SF 1642 from GE Bayer Silicone) | 5 |
| Polyethylene wax (weight-average MW 500) | 8 |

The silicone oil, the dimethiconol and the fluoro dimethicone are mixed together while hot until a uniform mixture is formed. The $C_{30}$-$C_{45}$ alkyl dimethicone is then added to the above mixture brought to 110°C. The polyethylene wax is then added portionwise until a uniform mixture is obtained. The mixture is cooled to 90-95°C and then poured into the

moulds, which are placed at -20°C for 30 minutes. Finally, the sticks are stripped from the moulds.

**Example 16: Measurement of the transfer resistance of the product comprising the composition of Example 11 as first composition and the composition of Example 15 as second composition**

The transfer index of the product comprising the composition of Example 11 as first composition and the composition of Example 15 as second composition is measured according to the method described above.

The transfer index of the product Lipfinity (shade 70), comprising a liquid lipstick and a colourless balm, is measured by proceeding as previously.

| Product whose transfer resistance is evaluated | Transfer value (in %) |
|---|---|
| Composition of Example 11 onto which is applied the composition of Example 15 | 4 ± 0.5 |
| Lipfinity product from Max Factor | 5 ± 0 |

**Example 17: Nail varnish kit**

| | |
|---|---|
| First composition | |
| Polymer of Example 1 | 23.8 g AM |
| Butyl acetate | 24.99 g |
| Isopropanol | 10.71 g |
| Hexylene glycol | 2.5 g |
| DC Red 7 Lake | 1 g |
| Hectorite modified with distearyl- dimethylbenzylammonium chloride (Bentone® 27V from Elementis) | 1.3 g |
| Ethyl acetate | qs 100 g |
| Second composition | |
| Nitrocellulose | 15 g |
| N-ethyl-o,p-toluenesulfonamide | 4 g |
| Tributyl acetyl citrate | 2 g |
| IPA | 6.5 g |
| qs (ethyl acetate, butyl acetate) | 100 g |

The first composition is applied to an individual's nails or false nails. Next, after drying, the second composition is applied over this first composition. The product obtained has noteworthy staying power, in particular noteworthy gloss fastness.

**Example 18: Nail varnish kit**

| | |
|---|---|
| First composition | |
| Polymer of Example 8 | 23.8 g AM |
| DC Red 7 Lake | 1 g |
| Hectorite modified with distearyl-dimethylbenzylammonium chloride (Bentone® 27V from Elementis) | 1.3 g |
| Ethyl acetate | qs 100 g |
| Second composition | |
| Nitrocellulose | 15 g |
| N-ethyl-o,p-toluenesulfonamide | 4 g |
| Tributyl acetyl citrate | 2 g |
| IPA | 6.5 g |
| qs (ethyl acetate, butyl acetate) | 100 g |

The first composition is applied to an individual's nails or false nails. Next, after drying, the second composition is applied over this first composition. The product obtained has noteworthy staying power, in particular noteworthy gloss fastness.

**Example 19: Mascara kit**

| | |
|---|---|
| First composition | |
| Beeswax | 6% |
| Paraffin wax | 13% |
| Hydrogenated jojoba oil | 2% |
| Water-soluble film-forming polymer | 3% |
| Triethanolamine stearate | 8% |
| Black pigment | 5% |
| Preserving agent | qs |
| Water | qs 100% |
| Second composition | |
| Beeswax | 8 g |
| Paraffin wax | 3 g |
| Carnauba wax | 6 g |
| Hectorite modified with distearyl-dimethylbenzylammonium chloride (Bentone® 38V from Elementis) | 5.3 g |
| Propylene carbonate | 1.7 g |
| Filler | 1 g |
| Pigments | 5 g |
| Polymer of Example 4 | 12 g AM |
| Isododecane | qs 100 |

The second composition may be applied to an individual's eyelashes. Next, after drying, the first composition is applied over this second composition. The product obtained has very good staying power.

**Example 20: Mascara kit**

| | |
|---|---|
| First composition | |
| Beeswax | 6% |
| Paraffin wax | 130% |
| Hydrogenated jojoba oil | 2% |
| Water-soluble film-forming polymer | 3% |
| Triethanolamine stearate | 8% |
| Black pigment | 5% |
| Preserving agent | qs |
| Water | qs 100% |
| Second composition | |
| Beeswax | 8 g |
| Paraffin wax | 3 g |
| Carnauba wax | 6 g |
| Hectorite modified with distearyl-dimethylbenzylammonium chloride (Bentone® 38V from Elementis) | 5.3 g |
| Propylene carbonate | 1.7 g |
| Filler | 1 g |
| Pigments | 5 g |

(continued)

| | |
|---|---|
| Second composition | |
| Polymer of Example 7 | 12 g AM |
| Isododecane | qs 100 |

The second composition may be applied to an individual's eyelashes. Next, after drying, the first composition is applied over this second composition. The product obtained has very good staying power.

**Example 21: Mascara as first composition**

| | |
|---|---|
| Beeswax | 8 g |
| Paraffin wax | 3 g |
| Carnauba wax | 6 g |
| Hectorite modified with distearyl-dimethylbenzylammonium chloride (Bentone® 38V from Elementis) | 5.3 g |
| Propylene carbonate | 1.7 g |
| Filler | 1 g |
| Pigments | 5 g |
| Polymer of Example 7 | 12 g AM |
| Isododecane | qs 100 |

**Examples 22 to 26: Mascara as first composition**

| Example | 22 | 23 | 24 | 25 | 26 |
|---|---|---|---|---|---|
| Polymer of Example 2 | 34.1 | | - | - | 45 |
| Polymer of Example 4 | | 34.1 | 45 | 45 | - |
| Triblock and radial block polymers | 3.4 | 3.4 | - | - | - |
| Octyldodecanol | 0.6 | 0.6 | - | 0.6 | - |
| Parleam oil | 1 | 1 | - | 1.4 | - |
| Phenyl trimethicone | 1 | 1 | - | 1.4 | - |
| PVP eicosene | 2.5 | 2.5 | - | 3.3 | - |
| Pigment | 2.5 | 2.5 | 10 | 3.3 | 10 |
| Filler | 0.5 | - | - | - | - |
| Mean gloss at 60° | - | 54.7 | 71.9 | 81.8 | 82.5 |

The gloss of the above examples is measured under the same conditions as described above.

**Example 27: Mascara kit**

First composition: that of Example 25

| | |
|---|---|
| Second composition | |
| Paraffin wax | 2.3 |
| Carnauba wax | 6.6 |
| Polyolefin wax | 2.1 |
| Beeswax | 8.3 |
| Modified hectorite | 5.8 |
| Silicone candelilla wax | 1 |
| Rice starch | 1.5 |
| Vinylpyrrolidone/eicosene copolymer | 2 |

(continued)

| | |
|---|---|
| Second composition | |
| Vinyl acetate/allyl stearate copolymer (Mexomer PQ from the company Chimex) | 2.7 |
| Polyvinyl laurate | 0.7 |
| Preserving agents | 0.1 |
| Polybutene | 1 |
| Propylene carbonate | 1.9 |
| Water | 7.6 |
| Ethanol | 2.7 |
| Black iron oxide | 4.2 |
| Isododecane | qs 100 |

A film of mascara composition of the second composition 300 micrometers thick is spread onto a glass plate. It is left to dry for 2 hours at room temperature (25°C) .
A film of the first composition 300 micrometers thick is then spread on top.
The assembly is left to dry for 24 hours at room temperature (25°C).
The mean gloss at 60° of the final film is then measured according to the method described above. The mean gloss of the product is 60.4.

## Claims

1. Cosmetic product comprising a first and a second composition, the first composition containing, in a cosmetically acceptable organic liquid medium at least one film-forming linear ethylenic block polymer, wherein said block polymer polymer comprises at least one first block and at least one second block that have different glass transition temperatures (Tg), wherein the first block of the polymer is chosen from:

   - a) a block with a Tg of greater than or equal to 40°C,
   - b) a block with a Tg of less than or equal to 20°C,
   - c) a block with a Tg of between 20 and 40°C, and

   the second block is chosen from a category a), b) or c) different from the first block, and wherein the first and second blocks are linked together via an intermediate segment, which is a random polymer, comprising at least one constituent monomer of the first block and at least one constituent monomer of the second block, and wherein said block polymer has a polydispersity index I of greater than 2,
   and the second composition, which is different from the first, comprising a cosmetically acceptable medium.

2. Cosmetic product according to any one of the preceding Claim, **characterized in that** the block polymer is free of styrene units.

3. Cosmetic product according to any one of the preceding Claim, **characterized in that** the block polymer is non-elastomeric.

4. Product according to one of the preceding claims, **characterized in that** the block polymer is an ethylenic polymer derived from aliphatic ethylenic monomers comprising a carbon-carbon double bond and at least one ester group -COO- or amide group -CON-.

5. Product according to one of the preceding claims, **characterized in that** the block polymer is not soluble at an active material content of at least 1% by weight in water or in a mixture of water and of linear or branched lower monoalcohols containing from 2 to 5 carbon atoms, without modification of pH, at room temperature (25°C).

6. Product according to one of the preceding claims, **characterized in that** the block polymer contains first and second blocks linked together via an intermediate segment comprising at least one constituent monomer of the first block and at least one constituent monomer of the second block.

7. Product according to one of the preceding claims, **characterized in that** the block polymer contains first and second

blocks that have different glass transition temperatures (Tg).

**8.** Product, according to any one of the p0receding claims, **characterized in that** the block polymer contains first and second blocks that are incompatible in the said organic liquid medium.

**9.** Product according to any one of the preceding Claims, **characterized in that** the block with a Tg of greater than or equal to 40°C is totally or partially derived from one or more monomers which are such that the homopolymer prepared from these monomers has a glass transition temperature of greater than or equal to 40°C.

**10.** Product according to the preceding claim, **characterized in that** the monomers whose corresponding homopolymer has a glass transition temperature of greater than or equal to 40°C are chosen from the following monomers:

methacrylates of formula $CH_2$ $C(CH_3)$-$COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2$ = CH-$COOR_2$ in which $R_2$ represents a $C_4$ to $C_{12}$ cycloalkyl group such as isobornyl acrylate or a tert-butyl group,
- (meth)acrylamides of formula:

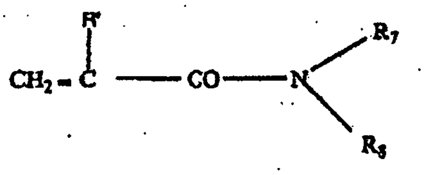

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl;
- and mixtures thereof.

**11.** Product according to Claim 9 or 10, **characterized in that** the monomers whose corresponding homopolymer has a glass transition temperature of greater,than or equal to 40°C are chosen from methyl methacrylates, isobutyl methacrylate and isobornyl (meth)acrylate, and mixtures thereof.

**12.** Product according to any one of the preceding Claims, **characterized in that** the block with a Tg of less than or equal to 20°C is totally or partially derived from one or more monomers which are such that the homopolymer prepared from these monomers has a glass transition temperature of less than or equal to 20°C.

**13.** Product according to Claim 12, **characterized in that** the monomers whose corresponding homopolymer has a glass transition temperature of less than or equal to 20°C are chosen from the following monomers:

- acrylates of formula $CH_2$ = $CHCOOR_3$, $R_3$ representing a linear or branched $C_1$ to $C_{12}$ unsubstituted alkyl group, with the exception of the tert-butyl group, in which one or more hetero atoms chosen from 0, N and S is (are) optionally intercalated,
- methacrylates of formula $CH_2$ = $C(CH_3)$-$COOR_4$, $R_4$ representing a linear or branched $C_6$ to $C_{12}$ unsubstituted alkyl group, in which one or more hetero atoms chosen from O, N and S is (are) optionally intercalated,
- vinyl esters of formula $R_5$-CO-O-CH = $CH_2$ in which $R_5$ represents a linear or branched $C_4$ to $C_{12}$ alkyl group,
- $C_4$ to $C_{12}$ alkyl vinyl ethers,
- N-($C_4$ to $C_{12}$)alkyl acrylamides, such as N-octylacrylamide,
- and mixtures thereof.

**14.** Product according to Claim 12 or 13, **characterized in that** the monomers whose corresponding homopolymer has a glass transition temperature of less than or equal to 20°Care chosen from alkyl acrylates whose alkyl chain contains from 1 to 10 carbon atoms, with the exception of the tert-butyl group.

**15.** Product according to any one of the preceding Claims 9, **characterized in that** the block with a Tg of between 20 and 40°C is totally or partially derived from one or more monomers which are such that the homopolymer prepared

from these monomers has a glass transition temperature of between 20 and 40°C.

16. Product according to any one of the preceding Claims, **characterized in that** the block with a Tg of between 20 and 40°C is totally or partially derived from monomers which are such that the corresponding homopolymer has a Tg of greater than or equal to 40°C and from monomers which are such that the corresponding homopolymer has a Tg of less than or equal to 20°C.

17. Product according to Claim 19 or 20, **characterized in that** the block with a Tg of between 20 and 40°C is partially derived from monomers chosen from methyl methacrylate, isobornyl acrylate and methacrylate, butyl acrylate and 2-ethylhexyl acrylate, and mixtures thereof.

18. Product according to one of Claims 1 to 14, **characterized in that** it comprises a block polymer comprising at least one first block and at least one second block, the first block having a glass transition temperature (Tg) of greater than or equal to 40°C and the second block having a glass transition temperature of less than or equal to 20°C.

19. Product according to the preceding claim, **characterized in that** the first block is totally or partially derived from one or more monomers which are such that the homopolymer prepared from these monomers has a glass transition temperature of greater than or equal to 40°C.

20. Product according to claim 19, **characterized in that** the first block is a copolymer derived from monomers which are such that the homopolymer prepared from these monomers has a glass transition temperature of greater than or equal to 40°C.

21. Product according to Claim 18 or 20, **characterized in that** the monomers whose corresponding homopolymer has a glass transition temperature of greater than or equal to 40°C are chosen from the following monomers:

- methacrylates of formula $CH_2$ = $C(CH_3)$-$COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2$ CH-$COOR_2$ in which $R_2$ represents a $C_4$ to $C_{12}$ cycloalkyl group such as isobornyl acrylate or a tert-butyl group, (meth)acrylamides of formula:

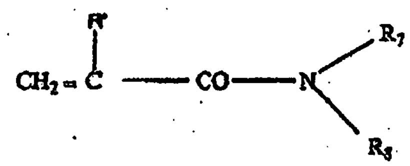

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl
- and mixtures thereof.

22. Product according to one of Claims 18 to 21, **characterized in that** the monomers whose corresponding homopolymer has a glass transition temperature of greater than or equal to 40°C are chosen from methyl methacrylate, isobutyl methacrylate and isobornyl (meth)acrylate, and mixtures thereof.

23. Product according to one of Claims 18 to 22, **characterized in that** the proportion of the first block ranges from 20% to 90% by weight, better still from 30% to 80% and even better still from 50% to 70% by weight of the polymer.

24. Product according to one of Claims 18 to 23, **characterized in that** the second block is totally or partially derived from one or more monomers which are such that the homopolymer prepared from these monomers has a glass transition temperature of less than or equal to 20°C.

25. Product according to one of Claims 18 to 24, **characterized in that** the second block is a homopolymer derived from monomers which are such that the homopolymer prepared from these monomers has a glass transition temperature of less than or equal to 20°C.

**26.** Product according to Claim 23 or 25, **characterized in that** the monomers whose corresponding homopolymer has a glass transition temperature of less than or equal to 20°C are chosen from the following monomers:

- acrylates of formula $CH_2 = CHCOOR_3$; $R_3$ representing a linear or branched $C_1$ to $C_{12}$ unsubstituted alkyl group, with the exception of the tert-butyl group, in which one or more hetero atoms chosen from O, N and S is (are) optionally intercalated,
- methacrylates of formula $CH_2 = C(CH_3)\text{-}COOR_4$, $R_4$ representing a linear or branched $C_6$ to $C_{12}$ unsubstituted alkyl group, in which one or more hetero atoms chosen from O, N and S is (are) optionally intercalated,
- vinyl esters of formula $R_5\text{-}CO\text{-}O\text{-}CH = CH_2$ in which $R_5$ represents a linear or branched $C_4$ to $C_{12}$ alkyl group,
- $C_4$ to $C_{12}$ alkyl vinyl ethers,
- N-($C_4$ to $C_{12}$) alkyl acrylamides, such as N-octylacrylamide,
- and mixtures thereof.

**27.** Product according to one of Claims 24 to 26, **characterized in that** the monomers whose corresponding homopolymer has a glass transition temperature of less than or equal to 20°C are chosen from alkyl acrylates whose alkyl chain contains from 1 to 10 carbon atoms, with the exception of the tert-butyl group.

**28.** Product according to one of Claims 18 to 27, **characterized in that** the proportion of the second block with a Tg of less than or equal to 20°C ranges from 5% to 75% by weight, better still from 15% to 50% and even better still from 25% to 45% by weight of the polymer.

**29.** Product according to one of Claims 1 to 14, **characterized in that** it comprises a block polymer comprising at least one first block and at least one second block, the first block having a glass transition temperature (Tg) of between 20 and 40°C and the second block having a glass transition temperature of less than or equal to 20°C or a glass transition temperature of greater than or equal to 40°C.

**30.** Product according to the preceding claim, **characterized in that** the first block with a Tg of between 20 and 40°C is totally or partially derived from one or more monomers which are such that the homopolymer prepared from these monomers has a glass transition temperature of between 20 and 40°C.

**31.** Product according to Claim 29 or 30, **characterized in that** the first block with a Tg of between 20 and 40°C is a copolymer derived from monomers which are such that the corresponding homopolymer has a Tg of greater than or equal to 40°C and from monomers which are such that the corresponding homopolymer, has a Tg of less than or equal to 20°C.

**32.** Product according to one of Claims 29 to 31, **characterized in that** the proportion of the first block with a Tg of between 20 and 40°C ranges from 10% to 85%, better still from 30% to 80% and even better still from 50% to 70% by weight of the polymer.

**33.** Product according to any one of Claims 29 to 31, **characterized in that** the second block has a Tg of greater than or equal to 40°C and is totally or partially derived from one or more monomers which are such that the homopolymer prepared from these monomers has a glass transition temperature of greater than or equal to 40°C.

**34.** Product according to any one of Claims 29 to 33, **characterized in that** the second block has a Tg of greater than or equal to 40°C and is a homopolymer derived from monomers which are such that the homopolymer prepared from these monomers has a glass transition temperature of greater than or equal to 40°C.

**35.** Product according to either of Claims 33 and 34, **characterized in that** the monomers whose corresponding homopolymer has a glass transition temperature of greater than or equal to 40°C are chosen from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2 = CH\text{-}COOR_2$ in which $R_2$ represents a $C_4$ to $C_{12}$ cycloalkyl group such as isobornyl acrylate or a tert-butyl group,

- (meth)acrylamides of formula:

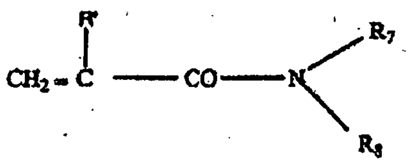

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl
- and mixtures thereof.

**36.** Product according to one of Claims 32 to 35, **characterized in that** the monomers whose corresponding homopolymer has a glass transition temperature of greater than or equal to 40°C are chosen from methyl methacrylate, isobutyl methacrylate and isobornyl (meth)acrylate, and mixtures thereof.

**37.** Product according to one of Claims 33 to 36, **characterized in that** the proportion of the second block with a Tg of greater than or equal to 40°C ranges from 10% to 85%, preferably from 20% to 70% and better still from 30% to 70% by weight of the polymer.

**38.** Product according to one of Claims 24 to 37, **characterized in that** the second block has a Tg of less than or equal to 20°C and is totally or partially derived from one or more monomers which are such that the homopolymer prepared from these monomers has a glass transition temperature of less than or equal to 20°C.

**39.** Product according to one of Claims 24 to 37, **characterized in that** the second block has a Tg of less than or equal to 20°C and is a homopolymer derived from monomers which are such that the homopolymer prepared from these monomers has a glass transition temperature of less than or equal to 20°C.

**40.** Product according to Claim 38 or 39, **characterized in that** the monomers whose corresponding homopolymer has a glass transition temperature of less than or equal to 20°C are chosen from the following monomers:

- acrylates of formula $CH_2 = CHCOOR_3$, $R_3$ representing a linear or branched $C_1$ to $C_{12}$ unsubstituted alkyl group, with the exception of the tert-butyl group, in which one or more hetero atoms chosen from O, N and S is (are) optionally intercalated,
- methacrylates of formula $CH_2 = C(CH_3)\text{-}COOR_4$ $R_4$ representing a linear or branched $C_6$ to $C_{12}$ unsubstituted alkyl group, in which one or more hetero atoms chosen from O, N and S is (are) optionally intercalated,
- vinyl esters of formula $R_5\text{-CO-O-CH} = CH_2$ in which $R_5$ represents a linear or branched $C_4$ to $C_{12}$ alkyl group,
- $C_4$ to $C_{12}$ alkyl vinyl ethers,
- N-($C_4$ to $C_{12}$)alkyl acrylamides, such as N-octylacrylamide,
- and mixtures thereof.

**41.** Product according to one of Claims 38 to 40, **characterized in that** the monomers whose homopolymers have glass transition temperatures of less than or equal to 20°C are chosen from alkyl acrylates whose alkyl chain contains from 1 to 10 carbon atoms, with the exception of the tert-butyl group.

**42.** Product according to one of Claims 38 to 41, **characterized in that** the proportion of the block with a glass transition temperature of greater than or equal to 40°C ranges from 20% to 90%, better still from 30% to 80% and even better still from 50% to 70% by weight of the polymer.

**43.** Product according to one of Claims 5 to 7 or any one of the preceding claims dependent thereon, **characterized in that** the first block and/or the second block comprises at least one additional monomer.

**44.** Product according to Claim 43, **characterized in that** the additional monomer is chosen from:

a) hydrophilic monomers such as:

- ethylenically unsaturated monomers comprising at least one.carboxylic or sulfonic acid function, for instance:

acrylic acid, methacrylic acid, crotonic acid; maleic anhydride, itaconic acid, fumaric acid, maleic acid, acrylamidopropanesulfonic acid, vinylbenzoic acid, vinylphosphoric acid, and salts thereof,

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof,
- methacrylates of formula $CH_2 = C(CH_3)\text{-}COOR_6$ in which $R_6$ represents a linear or branched alkyl group containing from 1 to 4 carbon atoms, such as a methyl, ethyl, propyl or isobutyl group, the said alkyl group being substituted with one or more substituents chosen from hydroxyl groups (for instance 2-hydroxypropyl methacrylate and 2-hydroxyethyl methacrylate) and halogen atoms (Cl, Br, I or F), such as trifluoroethyl methacrylate,
- methacrylates of formula $CH_2 = C(CH_3)\text{-}COOR_9$,
$R_9$ representing a linear or branched $C_6$ to $C_{12}$ alkyl group in which one or more hetero atoms chosen from O, N and S is (are) optionally intercalated, the said alkyl group being substituted with one or more substituents chosen from hydroxyl groups and halogen atoms (Cl, Br, I or F);
- acrylates of formula $CH_2 = CHCOOR_{10}$, $R_{10}$ representing a linear or branched $C_1$ to $C_{12}$ alkyl group substituted with one or more substituents chosen from hydroxyl groups and halogen atoms (Cl, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit 5 to 30 times, for example methoxy-POE, or $R_{10}$ represents a polyoxyethylenated group comprising from 5 to 30 ethylene oxide units, and

b) ethylenically unsaturated monomers comprising one or more silicon atoms, such as methacryloxypropyltrimethoxysilane and methacryloxypropyl-tris(trimethylsiloxy)silane,

- and mixtures thereof.

**45.** Product according to either of Claims 43 and 44, **characterized in that** each of the first and second blocks comprises at least one additional monomer chosen from acrylic acid, (meth)acrylic acid and trifluoroethyl methacrylate, and mixtures thereof.

**46.** Product according to either of Claims 43 and 44, **characterized in that** each of the first and second blocks comprises at least one monomer chosen from (meth)acrylic acid esters and optionally at least one additional monomer such as (meth)acrylic acid, and mixtures thereof

**47.** Product according to either of Claims 43 and 44, **characterized in that** each of the first and second blocks is totally derived from at least one monomer chosen from (meth)acrylic acid esters and optionally from at least one additional monomer such as (meth)acrylic acid, and mixtures thereof.

**48.** Product according to one of Claims 43 to 47, **characterized in that** the additional monomer(s) represent(s) from 1% to 30% by weight relative to the total weight of the first and/or second blocks.

**49.** Product according to Claim any one of the preceding claims, **characterized in that** the difference between the glass transition temperatures (Tg) of the first and second blocks is greater than 10°C, better still greater than 20°C, preferably greater than 30°C and better still greater than 40°C.

**50.** Product according to any one of the preceding Claims, **characterized in that** the block polymer has a polydispersity index of greater than or equal to 2.5 and preferably greater than or equal to 2.8.

**51.** Product according to Claim 50, **characterized in that** it has a polydispersity index of between 2.8 and 6.

**52.** Product according to one of the preceding claims, **characterized in that** the block polymer has a weight-average molecular mass (Mw) of less than or equal to 300 000

**53.** Product according to Claim 52, **characterized in that** the weight-average mass (Mw) ranges from 35 000 to 200 000 and better still from 45 000 to 150 000.

**54.** Product according to Claim 53, **characterized in that** the weight-average mass (Mn) is less than or equal to 70 000.

**55.** Product according to one of Claims 52 to 54, the weight-average mass (Mn) of which ranges from 10 000 to 60 000 and better still from 12 000 to 50 000.

**56.** Product according to one of the preceding claims, **characterized in that** it comprises from 0.1% to 60% by weight, preferably from 5% to 50% by weight and more preferably from 10% to 40% by weight of polymer active material.

**57.** Product according to one of the preceding claims, **characterized in that** the second composition is transparent.

**58.** Product according to one of the preceding claims, **characterized in that** the second composition contains less than 5%, preferably less than 2% and more preferably less than 1% of pigments.

**59.** Product according to one of the preceding claims, **characterized in that** the cosmetically acceptable medium of the second composition contains a non-volatile liquid phase comprising a hydrocarbon-based oil, a fluoro oil and/or a silicone oil.

**60.** Product according to one of the preceding claims, **characterized in that** the cosmetically acceptable medium of the second composition comprises a silicone polymer with a weight-average molecular mass of between 200 000 and 4 000 000 and more preferably between 200 000 and 2 000 000 g/mol, chosen from dimethiconols and polydimethylsiloxanes, and mixtures thereof.

**61.** Product according to one of the preceding claims, **characterized in that** the first composition and/or the second composition contains a colouring agent chosen from liposoluble dyes, water-soluble dyes, pigments and nacres, and mixtures thereof.

**62.** Product according to one of the preceding claims, **characterized in that** the first and/or the second composition is in the form of an anhydrous liquid, an anhydrous stick or an emulsion.

**63.** Product according to one of the preceding claims, **characterized in that** it is in the form of a foundation, a makeup rouge, an eyeshadow, a lipstick, a product with care properties, a mascara, an eyeliner, a nail varnish, a concealer product or a body makeup product.

**64.** Process for making up the skin and/or the lips and/or the integuments, which consists in applying to the skin, the lips and/or the integuments a first coat of a first composition comprising, in a first cosmetically acceptable medium, at least one film-forming ethylenic block polymer according to one of Claim 1 to 63 and a colouring agent, and then in applying, over all or part of the said first coat, a second coat of a second composition comprising a second cosmetically acceptable medium.

**65.** Process for making up the skin and/or the lips and/or the integuments, which consists in applying to the skin, the lips and/or the integuments a first coat of a first composition comprising, in a first cosmetically acceptable medium, at least one film-forming ethylenic block polymer according to one of Claim 1 to 63 and a colouring agent, in leaving the said first coat to dry and then in applying, over all or part of the first coat, a second coat of a second composition comprising a second cosmetically acceptable medium.

**66.** Process for making up the skin and/or the lips and/or the integuments, which consists in applying to the skin and/or the lips and/or the integuments a cosmetic product according to one of Claims 1 to 63.

**67.** Makeup kit comprising a product according to one of Claims 1 to 63.

**68.** Use of a cosmetic product comprising a first and a second composition, the first composition comprising, in a first cosmetically acceptable medium, at least one film-forming ethylenic block polymer according to one of Claim 1 to 63, and the second composition comprising a second cosmetically acceptable medium, at least one of the said compositions containing a colouring agent, to give the skin and/or the lips and/or the integuments a comfortable, glossy, transfer-resistant and/or migration-resistant makeup and/or a makeup with good colour fastness and/or with good gloss fastness.

**Patentansprüche**

1. Kosmetisches Produkt, das eine erste Zusammensetzung und eine zweite Zusammensetzung umfasst, wobei die erste Zusammensetzung in einem kosmetisch akzeptablen flüssigen organischen Medium mindestens ein filmbildendes lineares ethylenisches Blockpolymer enthält, wobei das Blockpolymer mindestens einen ersten Block und mindestens einen zweiten Block umfasst, die unterschiedliche Glasübergangstemperaturen (Tg) aufweisen, wobei der erste Block des Polymers ausgewählt ist unter:

    a) einem Block mit einer Tg von größer oder gleich 40 °C;
    b) einem Block mit einer Tg von kleiner oder gleich 20 °C;
    c) einem Block mit einer Tg zwischen 20 und 40 °C;

    und der zweite Block des Polymers aus einer vom den ersten Block verschiedenen Gruppe a), b) oder c) ausgewählt ist, wobei der erste Block und der zweite Block über ein Zwischensegment aneinandergebunden sind, bei dem es sich um ein statistisches Polymer handelt, das mindestens ein den ersten Block aufbauendes Monomer und mindestens ein den zweiten Block aufbauendes Monomer umfasst, wobei das Blockpolymer einen Polydispersitätsindex größer 2 aufweist,
    und die zweite Zusammensetzung, die von der ersten Zusammensetzung verschieden ist, ein kosmetisch akzeptables Medium enthält.

2. Kosmetisches Produkt nach einem der vorhergehenden Ansprüche, **dadurch gekennzeichnet, dass** das Blockpolymer keine Styroleinheiten enthält.

3. Kosmetisches Produkt nach einem der vorhergehenden Ansprüche, **dadurch gekennzeichnet, dass** das Blockpolymer nicht elastomer ist.

4. Produkt nach einem der vorhergehenden Ansprüche, **dadurch gekennzeichnet, dass** das Blockpolymer ein ethylenisches Polymer ist, das von aliphatischen ethylenischen Monomeren abgeleitet ist, die eine Kohlenstoff-Kohlenstoff-Doppelbindung und mindestens eine Estergruppe -COO- oder Amidgruppe - COM- aufweisen.

5. Produkt nach einem der vorhergehenden Ansprüche, **dadurch gekennzeichnet, dass** das Blockpolymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25°C) nicht löslich ist.

6. Produkt nach einem der vorhergehenden Ansprüche, **dadurch gekennzeichnet, dass** das Blockpolymer erste und zweite Blöcke aufweist, die über ein Zwischensegment aneinander gebunden sind, das mindestens ein den ersten Block aufbauendes Monomer und mindestens ein den zweiten Block aufbauendes Monomer umfasst.

7. Produkt nach einem der vorhergehenden Ansprüche, **dadurch gekennzeichnet, dass** das Blockpolymer erste und zweite Blöcke aufweist, die unterschiedliche Glasübergangstemperaturen (Tg) besitzen.

8. Produkt nach einem der vorhergehenden Ansprüche, **dadurch gekennzeichnet, dass** das Blockpolymer erste und zweite Blöcke aufweist, die in dem organischen flüssigen Medium inkompatibel sind.

9. Produkt nach einem der vorhergehenden Ansprüche, **dadurch gekennzeichnet, dass** der Block mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

10. Produkt nach einem der vorhergehenden Ansprüche, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

    - Methacrylaten der Formel $CH_2=C(CH_3)-COOR_1$, worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
    - Acrylaten der Formel $CH_2=CH-COOR_2$, worin $R_2$ eine Cycloalkylgruppe mit 4 bis 12 Kohlenstoffatomen, wie

Isobornylacrylat, oder tert-Butyl bedeutet,
- (Meth)acrylamiden der Formel:

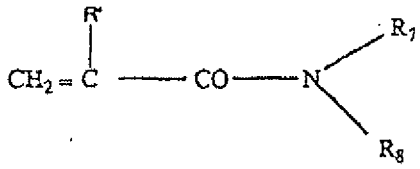

worin $R_7$ und $R_8$, die gleich oder verschieden sein können, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**11.** Produkt nach Anspruch 9 oder 10, **dadurch gekennzeichnet, dass** die Monomere, deren entsprechendes Homopolymer eine Glasübergangstemperatur von größer oder gleich 40 °C aufweist unter Methylmethacrylat, Isobutylacrylat und Isobornyl(meth)-acrylat und deren Gemischen ausgewählt sind.

**12.** Produkt nach einem der vorhergehenden Ansprüche, **dadurch gekennzeichnet, dass** der Block mit einer Tg von kleiner oder gleich 20 °C ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**13.** Produkt nach Anspruch 12, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2$=$CHCOOR_3$, worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel CH2=C($CH_3$)-$COOR_4$, worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$, worin $R_5$ eine lineare oder verzweigte Alkylgruppe mit 4 bis 12 Kohlenstoffatomen bedeutet,
- $C_4$-$_{12}$-Alkylvinylethern,
- N-Alkyl($C_{4-12}$)acrylamiden, wie N-Octylacrylamid,
- und deren Gemischen.

**14.** Produkt nach Anspruch 12 oder 13, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**15.** Produkt nach einem der vorhergehenden Ansprüche, **dadurch gekennzeichnet, dass** der Block mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**16.** Produkt nach einem der vorhergehenden Ansprüche, **dadurch gekennzeichnet, dass** der Block mit einer Tg zwischen 20 und 40°C ganz oder teilweise von einem oder mehreren Monomeren stammt, die so sind, dass das aus diesen Monomeren hergestellte Homopolymer eine Glasübergangstemperatur von größer oder gleich 40 °C aufweist, und von Monomeren abgeleitet ist, die so sind, dass das aus diesen Monomeren hergestellte Homopolymer eine Glasübergangstemperatur von kleiner oder gleich 20 °C aufweist.

**17.** Produkt nach Anspruch 19 oder 20, **dadurch gekennzeichnet, dass** der Block mit einer Tg zwischen 20 und 40°C von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**18.** Produkt nach einem der Ansprüche 1 bis 14, **dadurch gekennzeichnet, dass** es ein Blockpolymer umfasst, das mindestens einen ersten Block und mindestens einen zweiten Block aufweist, wobei der erste Block eine Glasübergangstemperatur (Tg) von größer oder gleich 40°C und der zweite Block eine Glasübergangstemperatur (Tg) von kleiner oder gleich 20°C aufweist.

**19.** Produkt nach dem vorhergehenden Anspruch, **dadurch gekennzeichnet, dass** der erste Block ganz oder teilweise von einem oder mehreren Monomeren stammt, die so sind, dass das aus diesen Monomeren hergestellte Homopolymer eine Glasübergangstemperatur von größer oder gleich 40°C aufweist,

**20.** Produkt nach Anspruch 19, **dadurch gekennzeichnet, dass** der erste Block ein Copolymer ist, das von Monomeren abgeleitet ist, die so sind, dass das aus diesen Monomeren hergestellte Homopolymer eine Glasübergangstemperatur von größer oder gleich 40 °C aufweist.

**21.** Produkt nach Anspruch 18 oder 20, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$, worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$, worin $R_2$ eine Cycloalkylgruppe mit 4 bis 12 Kohlenstoffatomen, wie Isobornylacrylat, oder die tert-Butylgruppe bedeutet,
- (Meth)acrylamiden der Formel:

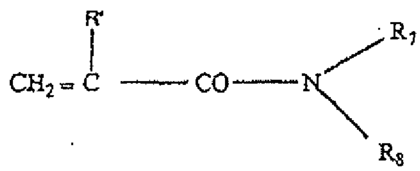

worin $R_7$ und $R_8$, die gleich oder verschieden sein können, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und
R'H oder Methyl bedeutet;
- und deren Gemischen.

**22.** Produkt nach einem der Ansprüche 18 bis 21, **dadurch gekennzeichnet, dass** die Monomere, deren entsprechendes Homopolymer eine Glasübergangstemperatur von größer oder gleich 40°C aufweist, unter Methylmethacrylat, Isobutylacrylat und Isobornyl(meth)acrylat und deren Gemischen ausgewählt sind.

**23.** Produkt nach einem der Ansprüche 18 bis 22, **dadurch gekennzeichnet, dass** der Mengenanteil des ersten Blocks im Bereich von 20 bis 90 Gew.-%, besser im Bereich von 30 bis 80 New.-% und noch besser im Bereich von 50 bis 70 Gew.-% des Polymers liegt.

**24.** Produkt nach einem der Ansprüche 18 bis 23, **dadurch gekennzeichnet, dass** der zweite Block ganz oder teilweise von einem oder mehreren Monomeren stammt, die so sind, dass das aus diesen Monomeren hergestellte Homopolymer eine Glasübergangstemperatur von kleiner oder gleich 20 ° aufweist.

**25.** Produkt nach einem der Ansprüche 18 bis 24, **dadurch gekennzeichnet, dass** der zweite Block ein Homopolymer ist, das von Monomeren abgeleitet ist, die so sind, dass das aus diesen Monomeren hergestellte Homopolymer eine Glasübergangstemperatur von kleiner oder gleich 20 ° aufweist.

**26.** Produkt nach Anspruch 24 oder 25, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2$=$CHCOOR_3$, worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2$=$C(CH_3)$-$CO0R_4$, worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$, worin $R_5$ eine lineare oder verzweigte Alkylgruppe mit 4 bis 12 Kohlenstoffatomen bedeutet,
- $C_{4-12}$-Alkylvinylethern,
- N-Alkyl($C_{4-12}$)acrylamiden, wie N-Octylacrylamid,
- und deren Gemischen.

**27.** Produkt nach einem der Ansprüche 24 bis 26, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**28.** Produkt nach einem der Ansprüche 18 bis 27, **dadurch gekennzeichnet, dass** der Mengenanteil des zweiten Blocks mit einer Tg von kleiner oder gleich 20 °C im Bereich von 5 bis 75 Gew.-%, besser im Bereich von 15 bis 50 Gew.-% und noch besser im Bereich von 25 bis 45 Gew.-% des Polymers liegt.

**29.** Produkt nach einem der Ansprüche 1 bis 14, **dadurch gekennzeichnet, dass** es ein Blockpolymer umfasst, das mindestens einen ersten Block und mindestens einen zweiten Block aufweist, wobei der erste Block eine Glasübergangstemperatur (Tg) zwischen 20 und 40°C und der zweite Block eine Glasübergangstemperatur von kleiner oder gleich 20°C oder eine Glasübergangstemperatur (Tg) von größerer oder gleich 40°C aufweist.

**30.** Produkt nach dem vorhergehenden Anspruch, **dadurch gekennzeichnet, dass** der erste Block mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**31.** Produkt nach Anspruch 29 oder 30, **dadurch gekennzeichnet, dass** der erste Block mit einer Tg zwischen 20 und 40°C ein Copolymer ist, das von Monomeren abgeleitet ist, die so sind, dass das aus diesen Monomeren hergestellte Homopolymer eine Glasübergangstemperatur von größer oder gleich 40 °C aufweist, und von Monomeren, die so sind, dass das aus diesen Monomeren hergestellte Homopolymer eine Tg von kleiner oder gleich 20 °C aufweist.

**32.** Produkt nach einem der Ansprüche 29 bis 31, **dadurch gekennzeichnet, dass** der Mengenanteil des ersten Blocks mit einer Tg zwischen 20 und 40°C im Bereich von 10 bis 85 Gew.-%, besser im Bereich von 30 bis 80 Gew.-% und noch besser im Bereich von 50 bis 70 Gew.-% liegt.

**33.** Produkt nach einem der Ansprüche 29 bis 31, **dadurch gekennzeichnet, dass** der zweite Block eine Tg von größer oder gleich 40 °C aufweist und ganz oder teilweise von Monomeren abgeleitet ist, die so sind, dass das aus diesen Monomeren hergestellte Homopolymer eine Glasübergangstemperatur von größer oder gleich 40 °C aufweist.

**34.** Produkt nach einem der Ansprüche 29 bis 33, **dadurch gekennzeichnet, dass** der zweite Block eine Tg von größer oder gleich 40 °C aufweist und ein Homopolymer ist, das ganz oder teilweise von Monomeren abgeleitet ist, die so sind, dass das aus diesen Monomeren hergestellte Homopolymer eine Glasübergangstemperatur von größer oder gleich 40 °C aufweist.

**35.** Produkt nach Anspruch 33 oder 34, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2$=$C(CH_3)$-$COOR_1$ worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2$=CH-$COOR_2$, worin $R_2$ eine Cycloalkylgruppe mit 4 bis 12 Kohlenstoffatomen, wie Isobornylacrylat, oder eine tert-Butylgruppe bedeutet,
- (Meth)acrylamiden der Formel:

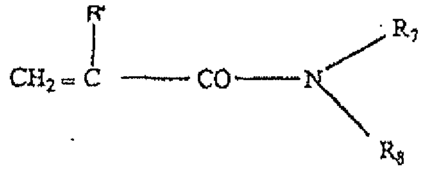

worin $R_7$ und $R_8$, die gleich oder verschieden sein können, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten oder $R_7$ H bedeutet und $R_8$ eine 1,1-Dimethyl-3-oxobutylgruppe ist, und R' H oder Methyl bedeutet;
- und deren Gemischen.

**36.** Produkt nach einem der Ansprüche 32 bis 35, **dadurch gekennzeichnet, dass** die Monomere, deren entsprechendes Homopolymer eine Glasübergangstemperatur von größer oder gleich 40 °C aufweist, unter Methylmethacrylat, Isobutylacrylat und Isobornyl(meth)acrylat und deren Gemischen ausgewählt sind.

**37.** Produkt nach einem der Ansprüche 33 bis 36, **dadurch gekennzeichnet, dass** der zweite Block mit einer Tg von größer oder gleich 40 °C in einem Mengenanteil im Bereich von 10 bis 85 Gew.-%, vorzugsweise im Bereich von 20 bis 70 Gew.-% und noch besser im Bereich von 30 bis 70 Gew.-% vorliegt.

**38.** Produkt nach einem der Ansprüche 24 bis 37, **dadurch gekennzeichnet, dass** der zweite Block eine Tg von kleiner oder gleich 20 °C aufweist und ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**39.** Produkt nach einem der Ansprüche 24 bis 37, **dadurch gekennzeichnet, dass** der zweite Block eine Tg von kleiner oder gleich 20°C aufweist und ein Homopolymer ist, das von Monomeren abgeleitet ist, die so sind, dass das aus diesen Monomeren hergestellte Homopolymer eine Glasübergangstemperatur von kleiner oder gleich 20°C aufweist.

**40.** Produkt nach Anspruch 38 oder 39, **dadurch gekennzeichnet, dass** die Monomere, deren entsprechendes Homopolymer eine Glasübergangstemperatur von kleiner oder gleich 20°C aufweist, unter den folgenden Monomeren ausgewählt sind:

- Acrylaten der Formel $CH_2$=$CHCOOR_3$, worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2$=$C(CH_3)$-$COOR_4$, worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$, worin $R_5$ eine lineare oder verzweigte Alkylgruppe mit 4 bis 12 Kohlenstoffatomen bedeutet,
- $C_{4-12}$-Alkylvinylethern,
- N-Alkyl($C_{4-12}$)acrylamiden, wie N-Octylacrylamid,
- und deren Gemischen.

**41.** Produkt nach einem der Ansprüche 38 bis 40, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**42.** Produkt nach einem der Ansprüche 38 bis 41, **dadurch gekennzeichnet, dass** der Anteil des Blocks mit einer Glasübergangstemperatur von größer oder gleich 40°C 20 bis 90 Gew.-%, besser 30 bis 80 Gew.-% und noch besser 50 bis 70 Gew.-% des Polymers ausmacht.

**43.** Produkt nach einem der Ansprüche 5 bis 7 oder einem von diesen abhängigen Ansprüchen, **dadurch gekennzeichnet, dass** der erste Block und/oder der zweite Block mindestens ein zusätzliches Monomer aufweisen.

**44.** Produkt nach Anspruch 43, **dadurch gekennzeichnet, dass** das zusätzliche Monomer ausgewählt ist unter:

a) hydrophilen Monomeren, wie:

- ethylenisch ungesättigten Monomeren, die mindestens eine Carbonsäurefunktion oder Sulfonsäurefunktion enthalten, wie beispielsweise Acrylsäure, Methacrylsäure, Crotonsäure, Maleinsäureanhydrid, Itaconsäure, Fumarsäure, Maleinsäure, Acrylamidopropansulfonsäure, Vinylbenzoesäure, Vinylphosphorsäure, und deren Salzen,
- ethylenisch ungesättigten Monomeren, die mindestens eine tertiäre Aminofunktion enthalten, wie beispielsweise 2-Vinylpyridin, 4-Vinylpyridin, Dimethylaminoethylmethacrylat, Diethylaminoethylmethacrylat und Dimethylaminopropylmethacrylamid, und deren Salzen,
- Methacrylaten der Formel $CH_2=C(CH_3)-COOR_6$, worin $R_6$ eine lineare oder verzweigte Alkylgruppe mit 1 bis 4 Kohlenstoffatomen bedeutet, wie Methyl, Ethyl, Propyl oder Isobutyl, wobei die Alkylgruppe mit einem oder mehreren Substituenten substituiert ist, die unter den Hydroxygruppen (z.B. 2-Hydroxypropylmethacrylat und 2-Hydroxyethylmethacrylat) und Halogenatomen (C1, Br, I oder F), wie Trifluorethylmethacrylat, ausgewählt sind,
- Methacrylaten der Formel $CH_2=C(CH_3)-COOR_9$, worin $R_9$ eine lineare oder verzweigte Alkylgruppe mit 6 bis 12 Kohlenstoffatomen bedeutet, in die optional ein oder mehrere Heteroatome, die unter O, N und S ausgewählt sind, eingebaut ist (sind), wobei die Alkylgruppe mit einem oder mehreren Substituenten substituiert ist, die unter den Hydroxygruppen und Halogenatomen (C1, Br, I oder F) ausgewählt sind,
- Acrylaten der Formel $CH_2=CHCO0R_{10}$, worin $R_{10}$ eine lineare oder verzweigte Alkylgruppe mit 1 bis 12 Kohlenstoffatomen bedeutet, die mit einem oder mehreren Substituenten substituiert ist, die unter den Hydroxygruppen und den Halogenatomen (C1, Br, I oder F) ausgewählt sind, wie 2-Hydroxypropylacrylat und 2-Hydroxyethylacrylat, oder $R_{10}$ eine Gruppe $C_{1-12}$-Alkyl-O-POE (Polyoxyethylen) bedeutet, wobei die Oxyethyleneinheit 5 bis 30 Mal wiederholt wird, beispielsweise Methoxy-POE, oder $R_{10}$ eine polyethoxylierte Gruppe bedeutet, die 5 bis 30 Ethylenoxideinheiten aufweist, und

b) ethylenisch ungesättigten Monomeren, die ein oder mehrere Siliciumatome enthalten, wie Methacryloxypropyltrimethoxysilan und Methacryloxypropyltris(trimethylsiloxy)silan,

und deren Gemischen.

**45.** Produkt nach Anspruch 43 oder 44, **dadurch gekennzeichnet, dass** der erste und der zweite Block jeder ein zusätzliches Monomer enthält, das unter Acrylsäure, (Meth)acrylsäure und Trifluorethylmethacrylat und deren Gemischen ausgewählt ist.

**46.** Produkt nach Anspruch 43 oder 44, **dadurch gekennzeichnet, dass** der erste und der zweite Block jeder ein zusätzliches Monomer enthält, das unter (Meth)acrylsäureestern und optional mindestens einem ergänzenden Monomer, wie (Meth)acrylsäure, und deren Gemischen ausgewählt ist.

**47.** Produkt nach Anspruch 43 oder 44, **dadurch gekennzeichnet, dass** der erste und der zweite Block jeder vollständig von mindestens einem Monomer abgeleitet ist, das unter (Meth)acrylsäureestern und optional mindestens einem ergänzenden Monomer, wie (Meth)acrylsäure, und deren Gemischen ausgewählt ist.

**48.** Produkt nach einem der Ansprüche 43 bis 47, **dadurch gekennzeichnet, dass** das oder die zusätzliche(n) Monomer (e) 1 bis 30 Gew.-%, bezogen auf das Gesamtgewicht des ersten und/oder zweiten Blocks, ausmachen.

**49.** Produkt nach einem der vorhergehenden Ansprüche, **dadurch gekennzeichnet, dass** die Differenz der Glasübergangstemperaturen (Tg) des ersten und des zweiten Blocks größer als 10 °C, besser größer als 20 °C, vorzugsweise größer als 30 °C und besonders bevorzugt größer als 40 °C ist.

**50.** Produkt nach einem der vorhergehenden Ansprüche, **dadurch gekennzeichnet, dass** das Blockpolymer einen Polydispersitätsindex von größer oder gleich 2,5 und vorzugsweise mindestens 2,8 aufweist.

**51.** Produkt nach Anspruch 50, **dadurch gekennzeichnet, dass** der Polydispersitätsindex im Bereich von 2,8 bis 6 liegt.

**52.** Produkt nach einem der vorhergehenden Ansprüche, **dadurch gekennzeichnet, dass** das Blockpolymer eine gewichtsmittlere Molmasse (Mw) von kleiner oder gleich 300 000 aufweist.

**53.** Produkt nach Anspruch 52, **dadurch gekennzeichnet, dass** die gewichtsmittlere Molmasse (Mw) im Bereich von

35 000 bis 200 000 und noch besser im Bereich von 45 000 bis 150 000 liegt.

**54.** Produkt nach Anspruch 53, **dadurch gekennzeichnet, dass** die gewichtsmittlere Molmasse (Mn) kleiner oder gleich 70 000 ist.

**55.** Produkt nach einem der Ansprüche 52 bis 54, dessen gewichtsmittlere Molmasse (Mn) im Bereich von 10 000 bis 60 000 und noch besser im Bereich von 12 000 bis 50 000 liegt.

**56.** Produkt nach einem der vorhergehenden Ansprüche, **dadurch gekennzeichnet, dass** es 0,1 bis 60 Gew.-%, vorzugsweise 5 bis 50 Gew.-% und noch bevorzugter 10 bis 40 Gew.-% des Polymermaterials enthält,

**57.** Produkt nach einem der vorhergehenden Ansprüche, **dadurch gekennzeichnet, dass** die zweite Zusammensetzung transparent ist.

**58.** Produkt nach einem der vorhergehenden Ansprüche, **dadurch gekennzeichnet, dass** die zweite Zusammensetzung weniger als 5 %, vorzugsweise weniger als 2 % und besonders bevorzugt weniger als 1 % Pigmente enthält.

**59.** Produkt nach einem der vorhergehenden Ansprüche, **dadurch gekennzeichnet, dass** das kosmetisch akzeptable Medium der zweiten Zusammensetzung eine nicht flüchtige flüssige Phase enthält, die ein Öl auf Kohlenwasserstoffbasis, ein fluoriertes Öl und/oder ein Siliconöl enthält.

**60.** Produkt nach einem der vorhergehenden Ansprüche, **dadurch gekennzeichnet, dass** das kosmetisch akzeptable Medium der zweiten Zusammensetzung ein Siliconpolymer mit einer gewichtsmittleren Molmasse im Bereich von 200 000 bis 4 000 000 g/mol und noch bevorzugter 200 000 bis 2 000 000 g/mol enthält, das unter den Dimethiconolen und Polydimethylsiloxanen und deren Gemischen ausgewählt ist.

**61.** Produkt nach einem der vorhergehenden Ansprüche, **dadurch gekennzeichnet, dass** die erste Zusammensetzung und/oder die zweite Zusammensetzung ein Farbmittel enthalten, das unter den fettlöslichen Farbstoffen, den wasserlöslichen Farbstoffen, den Pigmenten und Perlglanzpigmenten und deren Gemischen ausgewählt ist.

**62.** Produkt nach einem der vorhergehenden Ansprüche, **dadurch gekennzeichnet, dass** die erste Zusammensetzung und/oder die zweite Zusammensetzung in Form einer wasserfreien Flüssigkeit, eines wasserfreien Sticks oder einer Emulsion vorliegen.

**63.** Produkt nach einem der vorhergehenden Ansprüche, **dadurch gekennzeichnet, dass** es als Foundation, Wangenrouge, Lidschatten, Lippenstift, Produkt mit pflegenden Eigenschaften, Mascara, Eyeliner, Nagellack, Abdeckprodukt oder Produkt zum Schminkendes Körpers vorliegt.

**64.** Verfahren zum Schminken der Haut und/oder der Lippen und/ oder der Hautanhangsgebilde, das darin besteht, auf die Haut und/oder die Lippen und/oder die Hautanhangsgebilde eine erste Beschichtung einer ersten Zusammensetzung aufzubringen, die in einem ersten kosmetisch akzeptabeln Medium mindestens ein filmbildendes ethylenisches Blockpolymer nach einem der Ansprüche 1 bis 63 und ein Farbmittel enthält, und anschließend über einem Teil der ersten Beschichtung oder der ganzen ersten Beschichtung eine zweite Schicht einer zweiten Zusammensetzung aufzubringen, die ein zweites kosmetisch akzeptables Medium enthält.

**65.** Verfahren zum Schminken der Haut und/oder der Lippen und/ oder der Hautanhangsgebilde, das darin besteht, auf die Haut und/oder die Lippen und/oder die Hautanhangsgebilde eine erste Beschichtung einer ersten Zusammensetzung aufzubringen, die in einem ersten kosmetisch akzeptabeln Medium mindestens ein filmbildendes ethylenisches Blockpolymer nach einem der Ansprüche 1 bis 63 und ein Farbmittel enthält, die erste Beschichtung trocknen zu lassen und anschließend über einem Teil der ersten Beschichtung oder der ganzen ersten Beschichtung eine zweite Schicht einer zweiten Zusammensetzung aufzubringen, die zweites kosmetisch akzeptables Medium enthält.

**66.** Verfahren zum Schminken der Haut und/oder der Lippen und/ oder der Hautanhangsgebilde, das darin besteht, auf die Haut und/oder die Lippen und/oder die Hautanhangsgebilde ein kosmetisches Produkt nach einem der Ansprüche 1 bis 63 aufzubringen.

**67.** Make-up-Kit, das ein Produkt nach einem der Ansprüche 1 bis 63 enthält.

**68.** Verwendung eines kosmetischen Produktes, das eine erste Zusammensetzung und eine zweite Zusammensetzung umfasst, wobei die erste Zusammensetzung in einem ersten kosmetisch akzeptablen Medium mindestens ein filmbildendes ethylenisches Blockpolymer nach einem der Ansprüche 1 bis 63 enthält und die zweite Zusammensetzung ein zweites kosmetisch akzeptables Medium enthält, wobei mindestens eine dieser Zusammensetzungen ein Farbmittel enthält, um der Haut und/oder den Lippen und/oder den Hautanhangsgebilden ein angenehmes, strahlendes, transferbeständiges und/oder migrationsbeständiges Make-up und/oder Make-up mit hoher Farbfestigkeit und/oder großer Glanzbeständigkeit zu geben.

## Revendications

**1.** Produit cosmétique comprenant une première et une deuxième composition, la première composition contenant, dans un milieu liquide organique cosmétiquement acceptable, au moins un polymère bloc éthylénique linéaire filmogène, dans lequel ledit polymère bloc comprend au moins un premier bloc et au moins un deuxième bloc ayant des températures de transition vitreuse (Tg) différentes, où le premier bloc du polymère est choisi parmi :

a) un bloc ayant une Tg supérieure ou égale à 40°C ;
b) un bloc ayant une Tg inférieure ou égale à 20°C ;
c) un bloc ayant une Tg comprise entre 20 et 40°C, et le deuxième bloc est choisi parmi l'une des catégories a), b) ou c), différente du premier bloc, et où les premier et deuxième blocs sont reliés par un segment intermédiaire, qui est un polymère aléatoire, comprenant au moins un monomère constituant du premier bloc et au moins un monomère constituant du deuxième bloc, et où ledit polymère bloc possède un indice de polydispersité I supérieur à 2,

et la deuxième composition, qui est différente de la première, comprenant un milieu cosmétiquement acceptable.

**2.** Produit cosmétique selon l'une quelconque des revendications précédentes, **caractérisé en ce que** le polymère bloc est dépourvu de motifs styrène.

**3.** Produit cosmétique selon l'une quelconque des revendications précédentes, **caractérisé en ce que** le polymère bloc est non élastomère.

**4.** Produit selon l'une des revendications précédentes, **caractérisé en ce que** le polymère bloc est un polymère éthylénique dérivé de monomères éthyléniques aliphatiques comprenant une double liaison carbone-carbone et au moins un groupement ester -COO- ou groupement amide -CON-.

**5.** Produit selon l'une des revendications précédentes, **caractérisé en ce que** le polymère bloc n'est pas soluble à une teneur en matière active d'au moins 1% en poids dans l'eau ou dans un mélange d'eau et de mono-alcools inférieurs linéaires ou ramifiés contenant de 2 à 5 atomes de carbone, sans modification de pH, à température ambiante (25°C).

**6.** Produit selon l'une des revendications précédentes, **caractérisé en ce que** le polymère bloc contient un premier bloc et un deuxième bloc reliés par un segment intermédiaire comprenant au moins un monomère constituant du premier bloc et au moins un monomère constituant du deuxième bloc.

**7.** Produit selon l'une des revendications précédentes, **caractérisé en ce que** le polymère bloc contient un premier bloc et un deuxième bloc ayant des températures de transition vitreuse (Tg) différentes.

**8.** Produit selon l'une quelconque des revendications précédentes, **caractérisé en ce que** le polymère bloc contient un premier bloc et un deuxième bloc qui sont incompatibles dans ledit milieu liquide organique.

**9.** Produit selon l'une quelconque des revendications précédentes, **caractérisé en ce que** le bloc ayant une Tg supérieure ou égale à 40°C est totalement ou partiellement dérivé d'un ou plusieurs monomères qui sont tels que l'homopolymère préparé à partir de ces monomères a une température de transition vitreuse supérieure ou égale à 40°C.

**10.** Produit selon la revendication précédente, **caractérisé en ce que** les monomères dont l'homopolymère correspondant a une température de transition vitreuse supérieure ou égale à 40°C sont choisis parmi les monomères

suivantes :

les méthacrylates de formule $CH_2=C(CH_3)-COOR_1$, où $R_1$ représente un groupement alkyle non substitué linéaire ou ramifié contenant de 1 à 4 atomes de carbone, tel qu'un groupement méthyle, éthyle, propyle ou isobutyle, ou $R_1$ représente un groupement cycloalkyle de $C_4$ à $C_{12}$,
- des acrylates de formule $CH_2=CH-COOR_2$, où $R_2$ représente un groupement cycloalkyle de $C_4$ à $C_{12}$, tel qu'un acrylate d'isobornyle ou un groupement tertio-butyle,
- des (méth)acrylamides de formule

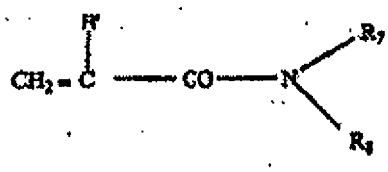

dans laquelle $R_7$ et $R_8$, qui peuvent être identiques ou différents, représentent chacun un atome d'hydrogène ou un groupement alkyle en $C_1$ à $C_{12}$ linéaire ou ramifié, tel qu'un groupement n-butyle, t-butyle, isopropyle, isohexyle, isooctyle ou isononyle : ou $R_7$ représente H et $R_8$ représente un groupement 1,1-diméthyl-3-oxobutyle, et R' désigne H ou méthyle ;
- et des mélanges de ceux-ci.

**11.** Produit selon la revendication 9 ou 10, **caractérisé en ce que** les monomères dont l'homopolymère correspondant a une température de transition vitreuse supérieure ou égale à 40°C sont choisis parmi le méthacrylate de méthyle, le méthacrylate d'isobutyle et le (méth)acrylate d'isobornyle, et des mélanges de ceux-ci.

**12.** Produit selon l'une quelconque des revendications précédentes, **caractérisé en ce que** le bloc ayant une Tg inférieure ou égale à 20°C est totalement ou partiellement dérivé d'un ou plusieurs monomères qui sont tels que l'homopolymère préparé à partir de ces monomères a une température de transition vitreuse inférieure ou égale à 20°C.

**13.** Produit selon la revendication 12, **caractérisé en ce que** les monomères dont l'homopolymère correspondant a une température de transition vitreuse inférieure ou égale à 20°C sont choisis parmi les monomères suivantes :

- les acrylates de formule $CH_2=CHCOOR_3$, $R_3$ représentant un groupement alkyle non substitué en $C_1$ à $C_{12}$ linéaire ou ramifié, à l'exception du groupement tertio-butyle, dans lequel un ou plusieurs hétéroatomes choisis parmi O, N et S sont éventuellement intercalés,
- les méthacrylates de formule $CH_2=C(CH_3)-COOR_4$, $R_4$ représentant un groupement alkyl non substitué en $C_6$ à $C_{12}$ linéaire ou ramifié, dans lequel un ou plusieurs hétéroatomes choisis parmi O, N et S sont éventuellement intercalés,
- les esters vinyliques de formule $R_5-CO-O-CH=CH_2$, où $R_5$ représente un groupement alkyle en $C_4$ à $C_{12}$ linéaire ou ramifié,
- les ($C_4$ à $C_{12}$) alkylvinyl éthers,
- les N-($C_4$ à $C_{12}$)alkylacrylamides, tels que le N-octylacrylamide,
- et des mélanges de ceux-ci.

**14.** Produit selon la revendication 12 ou 13, **caractérisé en ce que** les monomères dont l'homopolymère correspondant a une température de transition vitreuse inférieure ou égale à 20°C sont choisis parmi les acrylates d'alkyle dont la chaîne alkyle contient de 1 à 10 atomes de carbone, à l'exception du groupement tertio-butyle.

**15.** Produit selon l'une quelconque des revendications précédentes, **caractérisé en ce que** le bloc ayant une Tg comprise entre 20 et 40°C est totalement ou partiellement dérivé d'un ou plusieurs monomères qui sont tels que l'homopolymère préparé à partir de ces monomères a une température de transition vitreuse comprise entre 20 et 40°C.

**16.** Produit selon l'une quelconque des revendications précédentes, **caractérisé en ce que** le bloc ayant une Tg comprise entre 20 et 40°C est totalement ou partiellement dérivé d'un ou plusieurs monomères qui sont tels que l'homopolymère correspondant a une Tg supérieure ou égale à 40°C, et à partir de monomères qui sont tels que

l'homopolymère correspondant a une Tg inférieure ou égale à 20°C.

**17.** Produit selon la revendication 19 ou 20, **caractérisé en ce que** le bloc ayant une Tg comprise entre 20 et 40°C est partiellement dérivé de monomères choisis parmi le méthacrylate de méthyle, l'acrylate et méthacrylate d'isobornyle, l'acrylate de butyle et l'acrylate de 2-éthylhexyle, et des mélanges de ceux-ci.

**18.** Produit selon l'une des revendications 1 à 14, **caractérisé en ce qu'**il comprend un polymère bloc comprenant au moins un premier bloc et au moins un deuxième bloc, le premier bloc ayant une température de transition vitreuse (Tg) supérieure ou égale à 40°C et le deuxième bloc ayant une température de transition vitreuse inférieure ou égale à 20°C.

**19.** Produit selon la revendication précédente, **caractérisé en ce que** le premier bloc est totalement ou partiellement dérivé d'un ou plusieurs monomères qui sont tels que l'homopolymère préparé à partir de ces monomères a une température de transition vitreuse supérieure ou égale à 40°C.

**20.** Produit selon la revendication 19, **caractérisé en ce que** le premier bloc est un copolymère dérivé de monomères qui sont tels que l'homopolymère préparé à partir de ces monomères a une température de transition vitreuse supérieure ou égale à 40°C.

**21.** Produit selon la revendication 18 ou 20, **caractérisé en ce que** les monomères dont l'homopolymère correspondant a une température de transition vitreuse supérieure ou égale à 40°C sont choisis parmi les monomères suivantes :

- les méthacrylates de formule $CH_2=C(CH_3)-COOR_1$, où $R_1$ représente un groupement alkyle non substitué linéaire ou ramifié contenant de 1 à 4 atomes de carbone, tel qu'un groupement méthyle, éthyle, propyle ou isobutyle, ou $R_1$ représente un groupement cycloalkyle de $C_4$ à $C_{12}$,
- des acrylates de formule $CH_2=CH-COOR_2$, où $R_2$ représente un groupement cycloalkyle de $C_4$ à $C_{12}$, tel qu'un acrylate d'isobornyle ou un groupement tertio-butyle,
- des (méth)acrylamides de formule

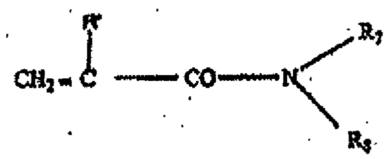

dans laquelle $R_7$ et $R_8$, qui peuvent être identiques ou différents, représentent chacun un atome d'hydrogène ou un groupement alkyle en $C_1$ à $C_{12}$ linéaire ou ramifié, tel qu'un groupement n-butyle, t-butyle, isopropyle, isohexyle, isooctyle ou isononyle ; ou $R_7$ représente H et $R_8$ représente un groupement 1,1-diméthyl-3-oxobutyle, et R' désigne H ou méthyle ;
- et des mélanges de ceux-ci.

**22.** Produit selon l'une des revendications 18 à 21, **caractérisé en ce que** les monomères dont l'homopolymère correspondant a une température de transition vitreuse supérieure ou égale à 40°C sont choisis parmi le méthacrylate de méthyle, le méthacrylate d'isobutyle et le (méth)acrylate d'isobornyle, et des mélanges de ceux-ci.

**23.** Produit selon l'une des revendications 18 à 22, **caractérisé en ce que** la proportion du premier bloc va de 20% à 90% en poids, mieux encore de 30% à 80%, et encore mieux de 50% à 70% en poids du polymère.

**24.** Produit selon l'une des revendications 18 à 23, **caractérisé en ce que** le deuxième bloc est totalement ou partiellement dérivé d'un ou plusieurs monomères qui sont tels que l'homopolymère préparé à partir de ces monomères a une température de transition vitreuse inférieure ou égale à 20°C.

**25.** Produit selon l'une des revendications 18 à 24, **caractérisé en ce que** le deuxième bloc est un homopolymère dérivé de monomères qui sont tels que l'homopolymère préparé à partir de ces monomères a une température de transition vitreuse inférieure ou égale à 20°C.

**26.** Produit selon la revendication 23 ou 25, **caractérisé en ce que** les monomères dont l'homopolymère correspondant a une température de transition vitreuse inférieure ou égale à 20°C sont choisis parmi les monomères suivantes :

- les acrylates de formule $CH_2$=CHCOOR$_3$, $R_3$ représentant un groupement alkyle non substitué en $C_1$ à $C_{12}$ linéaire ou ramifié, à l'exception du groupement tertio-butyle, dans lequel un ou plusieurs hétéroatomes choisis parmi O, N et S sont éventuellement intercalés,
- les méthacrylates de formule $CH_2$=C($CH_3$)-COOR$_4$, $R_4$ représentant un groupement alkyle non substitué en $C_6$ à $C_{12}$ linéaire ou ramifié, dans lequel un ou plusieurs hétéroatomes choisis parmi O, N et S sont éventuellement intercalés,
- les esters vinyliques de formule $R_5$-CO-O-CH=CH$_2$, où $R_5$ représente un groupement alkyle en $C_4$ à $C_{12}$ linéaire ou ramifié,
- les ($C_4$ à $C_{12}$)alkylvinyléthers,
- les N-($C_4$ à $C_{12}$)alkylacrylamides, tels que le N-octylacrylamide,
- et des mélanges de ceux-ci.

**27.** Produit selon l'une des revendications 24 à 26, **caractérisé en ce que** les monomères dont l'homopolymère correspondant a une température de transition vitreuse inférieure ou égale à 20°C sont choisis parmi les acrylates d'alkyle dont la chaîne alkyle contient de 1 à 10 atomes de carbone, à l'exception du groupement tertio-butyle.

**28.** Produit selon l'une des revendications 18 à 27, **caractérisé en ce que** la proportion du deuxième bloc ayant une Tg inférieure ou égale à 20°C va de 5% à 75% en poids, mieux encore de 15% à 50%, et encore mieux de 25% à 45% en poids du polymère.

**29.** Produit selon l'une des revendications 1 à 14, **caractérisé en ce qu'**il comprend un polymère bloc comprenant au moins un premier bloc et au moins un deuxième bloc, le premier bloc ayant une température de transition vitreuse (Tg) comprise entre 20 et 40°C et le deuxième bloc ayant une température de transition vitreuse inférieure ou égale à 20°C ou une température de transition vitreuse supérieure ou égale à 40°C.

**30.** Produit selon la revendication précédente, **caractérisé en ce que** le premier bloc ayant une Tg comprise entre 20 et 40°C est totalement ou partiellement dérivé d'un ou plusieurs monomères qui sont tels que l'homopolymère préparé à partir de ces monomères a une température de transition vitreuse comprise entre 20 et 40°C.

**31.** Produit selon la revendication 29 ou 30, **caractérisé en ce que** le premier bloc ayant une Tg comprise entre 20 et 40°C est un copolymère dérivé de monomères qui sont tels que l'homopolymère correspondant a une Tg supérieure ou égale à 40°C, et de monomères qui sont tels que l'homopolymère correspondant a une Tg inférieure ou égale à 20°C.

**32.** Produit selon l'une des revendications 29 à 31, **caractérisé en ce que** la proportion du premier bloc ayant une Tg comprise entre 20 et 40°C va de 10% à 85%, mieux encore de 30% à 80%, et encore mieux de 50% à 70% en poids du polymère.

**33.** Produit selon l'une quelconque des revendications 29 à 31, **caractérisé en ce que** le deuxième bloc a une Tg supérieure ou égale à 40°C et est totalement ou partiellement dérivé d'un ou plusieurs monomères qui sont tels que l'homopolymère préparé à partir de ces monomères a une température de transition vitreuse supérieure ou égale à 40°C.

**34.** Produit selon l'une quelconque des revendications 29 à 33, **caractérisé en ce que** le deuxième bloc a une Tg supérieure ou égale à 40°C et est un homopolymère dérivé de monomères qui sont tels que l'homopolymère préparé à partir de ces monomères a une température de transition vitreuse supérieure ou égale à 40°C.

**35.** Produit selon l'une ou l'autre des revendications 33 et 34, **caractérisé en ce que** les monomères dont l'homopolymère correspondant a une température de transition vitreuse supérieure ou égale à 40°C sont choisis parmi les monomères suivants :

- les méthacrylates de formule $CH_2$=C($CH_3$)-COOR$_1$, où $R_1$ représente un groupement alkyle non substitué linéaire ou ramifié contenant de 1 à 4 atomes de carbone, tel qu'un groupement méthyle, éthyle, propyle ou isobutyle, ou $R_1$ représente un groupement cycloalkyle de $C_4$ à $C_{12}$,
- des acrylates de formule $CH_2$=CH-COOR$_2$, où $R_2$ représente un groupement cycloalkyle de $C_4$ à $C_{12}$, tel qu'un

acrylate d'isobornyle ou un groupement tertio-butyle,
- des (méth)acrylamides de formule

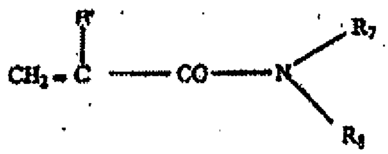

dans laquelle $R_7$ et $R_8$, qui peuvent être identiques ou différents, représentent chacun un atome d'hydrogène ou un groupement alkyle en $C_1$ à $C_{12}$ linéaire ou ramifié, tel qu'un groupement n-butyle, t-butyle, isopropyle, isohexyle, isooctyle ou isononyle ; ou $R_7$ représente H et $R_8$ représente un groupement 1,1-diméthyl-3-oxobutyle, et R' désigne H ou méthyle ;
- et des mélanges de ceux-ci.

**36.** Produit selon l'une des revendications 32 à 35, **caractérisé en ce que** les monomères dont l'homopolymère correspondant a une température de transition vitreuse supérieure ou égale à 40°C sont choisis parmi le méthacrylate de méthyle, le méthacrylate d'isobutyle et le (méth)acrylate d'isobornyle, et des mélanges de ceux-ci.

**37.** Produit selon l'une des revendications 33 à 36, **caractérisé en ce que** la proportion du deuxième bloc ayant une Tg supérieure ou égale à 40°C va de 10% à 85%, préférablement de 20% à 70%, et mieux encore de 30% à 70% en poids du polymère.

**38.** Produit selon l'une des revendications 24 à 37, **caractérisé en ce que** le deuxième bloc a une Tg inférieure ou égale à 20°C et est totalement ou partiellement dérivé d'un ou plusieurs monomères qui sont tels que l'homopolymère préparé à partir de ces monomères a une température de transition vitreuse inférieure ou égale à 20°C.

**39.** Produit selon l'une des revendications 24 à 37, **caractérisé en ce que** le deuxième bloc a une Tg inférieure ou égale à 20°C et est un homopolymère dérivé de monomères qui sont tels que l'homopolymère préparé à partir de ces monomères a une température de transition vitreuse inférieure ou égale à 20°C.

**40.** Produit selon la revendication 38 ou 39, **caractérisé en ce que** les monomères dont l'homopolymère correspondant a une température de transition vitreuse inférieure ou égale à 20°C sont choisis parmi les monomères suivants :

- les acrylates de formule $CH_2$=$CHCOOR_3$, $R_3$ représentant un groupement alkyle non substitué en $C_1$ à $C_{12}$ linéaire ou ramifié, à l'exception du groupement tertio-butyle, dans lequel un ou plusieurs hétéroatomes choisis parmi O, N et S sont éventuellement intercalés,
- les méthacrylates de formule $CH_2$=$C(CH_3)$-$COOR_4$, $R_4$ représentant un groupement alkyle non substitué en $C_6$ à $C_{12}$ linéaire ou ramifié, dans lequel un ou plusieurs hétéroatomes choisis parmi O, N et S sont éventuellement intercalés,
- les esters vinyliques de formule $R_5$-CO-O-CH=$CH_2$, où $R_5$ représente un groupement alkyle en $C_4$ à $C_{12}$ linéaire ou ramifié,
- les ($C_4$ à $C_{12}$)alkylvinyléthers,
- les N-($C_4$ à $C_{12}$)alkylacrylamides, tels que le N-octylacrylamide,
- et des mélanges de ceux-ci.

**41.** Produit selon l'une des revendications 38 à 40, **caractérisé en ce que** les monomères dont l'homopolymère correspondant a une température de transition vitreuse inférieure ou égale à 20°C sont choisis parmi les acrylates d'alkyle dont la chaîne alkyle contient de 1 à 10 atomes de carbone, à l'exception du groupement tertio-butyle.

**42.** Produit selon l'une des revendications 38 à 41, **caractérisé en ce que** la proportion du bloc ayant une température de transition vitreuse supérieure ou égale à 40°C va de 20% à 90%, mieux encore de 30% à 80%, et encore mieux de 50% à 70% en poids du polymère.

**43.** Produit selon l'une des revendications 5 à 7 ou selon l'une quelconque des revendications précédentes en étant dépendante, **caractérisé en ce que** le premier bloc et/ou le deuxième bloc comprend au moins un monomère supplémentaire.

**44.** Produit selon la revendication 43, **caractérisé en ce que** le monomère supplémentaire est choisi parmi :

a) des monomères hydrophiles, tels que

- des monomères éthyléniquement insaturés comprenant au moins une fonction acide carboxylique ou sulfonique, par exemple :

l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique, et les sels de ceux-ci,

- des monomères éthyléniquement insaturés comprenant au moins une fonction amine tertiaire, par exemple la 2-vinylpyridine, la 4-vinylpyridine, le méthacrylate de diméthylaminoéthyle, le méthacrylate de diéthylaminoéthyle, et le diméthylaminopropylméthacrylamide, et les sels de ceux-ci,
- les méthacrylates de formule $CH_2=C(CH_3)-COOR_6$, où $R_6$ représente un groupement alkyle linéaire ou ramifié contenant de 1 à 4 atomes de carbone, tel qu'un groupement méthyle, éthyle, propyle ou isobutyle, ledit groupement alkyle étant substitué par un ou plusieurs substituants choisis parmi des groupements hydroxyle (par exemple le méthacrylate de 2-hydroxypropyle et le méthacrylate de 2-hydroxyéthyle) et des atomes d'halogène (C1, Br, I ou F), tels que le méthacrylate de trifluoroéthyle,
- les méthacrylates de formule $CH_2=C(CH_3)-COOR_9$, $R_9$ représentant un groupement alkyle en $C_6$ à $C_{12}$ linéaire ou ramifié dans lequel un ou plusieurs hétéroatomes choisis parmi O, N et S sont éventuellement intercalés, ledit groupement alkyle étant substitué par un ou plusieurs substituants choisis parmi des groupements hydroxyle et des atomes d'halogène (Cl, Br, I ou F),
- les acrylates de formule $CH_2=CHCOOR_{10}$, $R_{10}$ représentant un groupement alkyle en $C_1$ à $C_{12}$ linéaire ou ramifié substitué par un ou plusieurs substituants choisis parmi des groupements hydroxyle et des atomes d'halogène (Cl, Br, I ou F), tels que l'acrylate de 2-hydroxypropyle et l'acrylate de 2-hydroxyéthyle, ou $R_{10}$ représente un ($C_1$ à $C_{12}$)alkyl-O-POE (polyoxyéthylène) avec une répétition du motif oxyéthylène de 5 à 30 fois, par exemple méthoxy-POE, ou
$R_{10}$ représente un groupement polyoxyéthyléné comprenant de 5 à 30 motifs d'oxyde d'éthylène, et

b) des monomères éthyléniquement insaturés comprenant un ou plusieurs atomes de silicium, tels que le méthacryloxypropyltriméthoxysilane et le méthacryloxypropyltris(triméthylsiloxy)silane,

- et des mélanges de ceux-ci.

**45.** Produit selon l'une ou l'autre des revendications 43 et 44, **caractérisé en ce que** chacun parmi le premier et le deuxième bloc comprend au moins un monomère supplémentaire choisi parmi l'acide acrylique, l'acide (méth) acrylique et le méthacrylate de trifluoroéthyle, et des mélanges de ceux-ci.

**46.** Produit selon l'une ou l'autre des revendications 43 et 44, **caractérisé en ce que** chacun parmi le premier et le deuxième bloc comprend au moins un monomère choisi parmi les esters de l'acide (méth)acrylique et éventuellement au moins un monomère supplémentaire tel que l'acide (méth)acrylique, et des mélanges de ceux-ci.

**47.** Produit selon l'une ou l'autre des revendications 43 et 44, **caractérisé en ce que** chacun parmi le premier et le deuxième bloc est totalement dérivé à partir d'au moins un monomère choisi parmi les esters de l'acide (méth) acrylique et éventuellement à partir d'au moins un monomère supplémentaire tel que l'acide (méth)acrylique, et des mélanges de ceux-ci.

**48.** Produit selon l'une des revendications 43 à 47, **caractérisé en ce que** le ou les monomères supplémentaires représentent de 1% à 30% en poids par rapport au poids total du premier et/ou du deuxième bloc.

**49.** Produit selon l'une quelconque des revendications précédentes, **caractérisé en ce que** la différence entre les températures de transition vitreuse (Tg) du premier et du deuxième bloc est supérieure à 10°C, mieux encore supérieure à 20°C, préférablement supérieure à 30°C et encore mieux supérieure à 40°C.

**50.** Produit selon l'une quelconque des revendications précédentes, **caractérisé en ce que** le polymère bloc a un indice de polydispersité supérieur ou égal à 2,5, et préférablement supérieur ou égal à 2,8.

**51.** Produit selon la revendication 50, **caractérisé en ce qu'**il a un indice de polydispersité compris entre 2,8 et 6.

**52.** Produit selon l'une quelconque des revendications précédentes, **caractérisé en ce que** le polymère bloc a une masse moléculaire moyenne en poids (Mw) inférieure ou égale à 300 000.

**53.** Produit selon la revendication 52, **caractérisé en ce que** la masse moyenne en poids (Mw) va de 35 000 à 200 000 et mieux encore de 45 000 à 150 000.

**54.** Produit selon la revendication 53, **caractérisé en ce que** la masse moyenne en poids (Mn) est inférieure ou égale à 70 000.

**55.** Produit selon l'une des revendications 52 à 54, dont la masse moyenne en poids (Mn) va de 10 000 à 60 000 et mieux encore de 12 000 à 50 000.

**56.** Produit selon l'une des revendications précédentes, **caractérisé en ce qu'**il comprend de 0,1% à 60% en poids, préférablement de 5% à 50% en poids et plus préférablement de 10% à 40% en poids de matière active polymère.

**57.** Produit selon l'une des revendications précédentes, **caractérisé en ce que** la deuxième composition est transparente.

**58.** Produit selon l'une des revendications précédentes, **caractérisé en ce que** la deuxième composition contient moins de 5%, préférablement moins de 2% et plus préférablement moins de 1% de pigments.

**59.** Produit selon l'une des revendications précédentes, **caractérisé en ce que** le milieu cosmétiquement acceptable de la deuxième composition contient une phase liquide non volatile comprenant une huile à base d'hydrocarbures, une huile fluorée et/ou une huile siliconée.

**60.** Produit selon l'une des revendications précédentes, **caractérisé en ce que** le milieu cosmétiquement acceptable de la deuxième composition comprend un polymère de silicone ayant une masse moléculaire moyenne en poids comprise entre 200 000 et 4 000 000 et plus préférablement entre 200 000 et 2 000 000 g/mol, choisi parmi les diméthiconols et les polydiméthylsiloxanes, et des mélanges de ceux-ci.

**61.** Produit selon l'une des revendications précédentes, **caractérisé en ce que** la première composition et/ou la deuxième composition contient un agent de coloration choisi parmi les colorants liposolubles, les colorants hydrosolubles, les pigments et les nacres, et des mélanges de ceux-ci.

**62.** Produit selon l'une des revendications précédentes, **caractérisé en ce que** la première composition et/ou la deuxième composition est sous la forme d'un liquide anhydre, d'un stick anhydre ou d'une émulsion.

**63.** Produit selon l'une des revendications précédentes, **caractérisé en ce qu'**il se trouve sous la forme d'un fond de teint, d'un fard à joues, d'un fard à paupières, d'un rouge à lèvres, d'un produit ayant des propriétés de soin, d'un mascara, d'un eye-liner, d'un vernis à ongles, d'un correcteur de teint ou d'un produit de maquillage corporel.

**64.** Procédé de maquillage de la peau et/ou des lèvres et/ou des téguments, consistant à appliquer à la peau, aux lèvres et/ou aux téguments, une première couche d'une première composition comprenant, dans un premier milieu cosmétiquement acceptable, au moins un polymère bloc éthylénique filmogène selon l'une des revendications 1 à 63 et un agent de coloration, puis à appliquer, sur l'ensemble ou une partie de ladite première couche, une deuxième couche d'une deuxième composition comprenant un deuxième milieu cosmétiquement acceptable.

**65.** Procédé de maquillage de la peau et/ou des lèvres et/ou des téguments, consistant à appliquer à la peau, aux lèvres et/ou aux téguments, une première couche d'une première composition comprenant, dans un premier milieu cosmétiquement acceptable, au moins un polymère bloc éthylénique filmogène selon l'une des revendications 1 à 63 et un agent de coloration, à laisser ladite première couche sécher, puis à appliquer, sur l'ensemble ou une partie de la première couche, une deuxième couche d'une deuxième composition comprenant un deuxième milieu cosmétiquement acceptable.

**66.** Procédé de maquillage de la peau et/ou des lèvres et/ou des téguments, consistant à appliquer à la peau et/ou aux lèvres et/ou aux téguments, un produit cosmétique selon l'une des revendications 1 à 63.

**67.** Kit de maquillage comprenant un produit selon l'une des revendications 1 à 63.

**68.** Utilisation d'un produit cosmétique comprenant une première et une deuxième composition, la première composition comprenant, dans un premier milieu cosmétiquement acceptable, au moins un polymère bloc éthylénique filmogène selon l'une des revendications 1 à 63, et la deuxième composition comprenant un deuxième milieu cosmétiquement acceptable, au moins l'une desdites compositions comprenant un agent de coloration, afin de donner à la peau et/ou aux lèvres et/ou aux téguments, un maquillage confortable, brillant, résistant au transfert et/ou résistant à la migration et/ou un maquillage ayant une bonne solidité de la couleur et/ou ayant une bonne solidité du brillant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2823101 A
- JP 05221829 A
- WO 9717057 A
- US 6001374 A
- WO 02067877 A
- EP 1440680 A
- WO 03046032 A
- US 4152416 A
- FR 0209246
- EP 0667146 A


### Non-patent literature cited in the description


- **Buzin et al.** calorimetric study of block-copolymer of poly(n-butyl acrylate) and gradient poly(n-butyl acrylate-co-methyl-methacrylate). *Polymer,* 2002, vol. 43, 5563-5569
- Polymer Handbook. John Wiley, 1989